# EUROPEAN PATENT SPECIFICATION

(11) **EP 1 562 974 B1**
(45) Date of publication and mention of the grant of the patent: **21.02.2007**
(21) Application number: 03716433.2
(22) Date of filing: 21.03.2003
(51) Int. Cl.: C07J 1/00, C07J 21/00

(54) **PROCESSES FOR PREPARING C-7 SUBSTITUTED 5-ANDROSTENES**
VERFAHREN ZUR HERSTELLUNG C-7 SUBSTITUIERTER 5-ANDROSTENE
PROCEDES DE PREPARATION DE 5-ANDROSTENES SUBSTITUES EN C-7

(30) Priority: 07.11.2002 US 424488 P
(43) Date of publication of application: 17.08.2005
(73) Proprietor: Pharmacia & Upjohn Company LLC, Kalamazoo, MI 49001 (US)
(72) Inventor: WUTS, Peter, GM, Mattawan, MI 49071 (US)
(74) Representative: Laurent, Claire
(86) International application number: PCT/US2003/007284
(87) International publication number: WO 2004/043986

(56) References cited:
- WO-A-97/21720
- US-A- 5 227 375
- NEGI A S ET AL: "An approach towards the development of progesterone antagonists: Synthesis of 7alpha/7beta-aryl androstene derivatives" STEROIDS: STRUCTURE, FUNCTION, AND REGULATION, ELSEVIER SCIENCE PUBLISHERS, NEW YORK, NY, US, vol. 60, no. 6, 1 June 1995 (1995-06-01), pages 470-472, XP004026514 ISSN: 0039-128X

## Description

### BACKGROUND OF THE INVENTION

Certain C-7 substituted steroids, for example eplerenone, are well known for their aldosterone antagonist activity and are thus useful in the treatment and prevention of diseases of the circulatory system. Eplerenone is the subject of several patents and applications, for example, U.S. Pat. Nos. 4,559,332 and 5,981,744 and International Publications WO98/25948 and WO97/21720. However, the advent of new and expanded clinical uses for eplerenone create a need for improved processes for the manufacture of this and other related steroids. A major obstacle to the efficient synthesis of eplerenone and related steroid compounds is the introduction of a carboxy group at C-7 or functionality which can be transformed into a carboxy group.

Allylic derivatives, and in particular allylic acetates, benzoates, pivalates and the like are known to react with nucleophilic reagents under the influence of a Lewis acid in a process called "allylation" as has been described. The allylation reaction has been applied to a number of substrates. For example, glycals have been shown to yield allyl glycosides, glycosyl cyanides and gylcosal azides upon allylation (Yadav, J.S., et.al., Tetrahedron Lett., 2001, 42, 4057. Allylic acetates and carbonates give the corresponding cyanides (Yasushi, T., et.al., J. Org. Chem., 1993, 58, 16). Electron rich aromatic and heteroaromatics give the corresponding allylated products (Malkov, A.V., et.al., J. Org. Chem., 1999,64,2751). The allylation reaction however, has not heretofore been applied to steroids to give 7-substituted steroids useful for the conversion to 7-carboxy substituted steroids such as eplereneone. 3,17-diacetoxy-7-hydroxyandrost-5-ene, or the corresponding 7-methane sulfonates, has been reacted with phenol and anisole using the harsh catalyst aluminum chloride (Negi, A.S., et.al., Steroids, 1995, 60, 470). The resultant 7-aryl derivatives are obtained in low yield as a mixture of C-7 epimers. Further, the 7-aryl derivatives would be difficult at best for use in preparation of 7-carboxy substituted steroids.

### SUMMARY OF THE INVENTION

This invention relates to processes for the preparation of novel 7-carboxy substituted steroid compounds of Formula I, 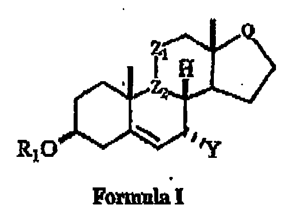 wherein
R₁ is -COR₂;
R₂ is C₁-C₆ alkyl or C₁-C₆ alkoxy;
Z₁ is CH₂ or 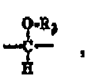 wherein
   OR₃ is in the α configuration;
   R₃ is H or -COR₂;
Z₂ is CH ; or
Z₁ and Z₂ may be taken together to form a carbon double bond;
Qis 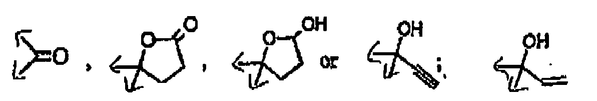
Y is -CN, -CH₂-CH=CH₂, 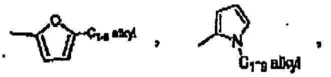 CHR₄C(O)Ar, CHR₄C(O)C₁₋₆alkyl, CHR₄C(O)XAr, or CHR₄ C(O)XC_{1-6alkyl};
   where
   R₄ = O C₁₋₆alkyl or aryl
   X=O or S

These novel intermediates are useful in the preparation of 7-carboxy substituted steroid compounds, and particularly, the invention is directed to novel and advantageous methods for the preparation of 9,11-α-epoxy-17-α-hydroxy-3-oxopregn-4-ene-α-21-dicarboxylic acid, γ-lactone, methyl ester (eplerenone; epoxymexrenone).

A key step in the processes of the present invention is reacting a novel steroid intermediate of Formula II, 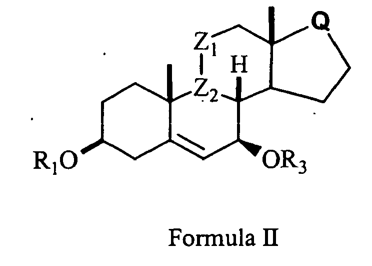 wherein
R₁ and R₃ are independently selected from H, C(O)OR₂ or COR₂ and at least one of R₁ or R₂ is C(O)OR₂ or COR₂;
Z₁, Z₂, R₂ and Q are as for Formula I;
with a nucleophilic reagent selected from the group of C₁₋₄-trialkylsilylcyanides, C₁₋₄-trialkylsilylenolethers, C₁₋₄- trialkylsilyloxyketenethioacetals (i.e. RCH=C(OSiR_{C1-C6alkyl})SR_{C1-6alkyl}), allyltri-C₁₋₄-alkylsilanes, allyltri-C₁₋₄-alkylstannanes, 2-C₁₋₄-alkylfurans and 2-C₁₋₄-alkylpyrroles in the presence of a Lewis acid catalyst.

Within a compound of Formula II resides the structural element of an allylic alcohol derivative at C-5, -C6, - C7- OR₃. The novel synthesis schemes which take advantage of the "allylation" reaction are described in detail in the Description of Embodiments.

### DESCRIPTION OF THE EMBODIMENTS

In one embodiment of the invention, there is provided a process for preparing 7-substituted steroid compounds of Formula I, 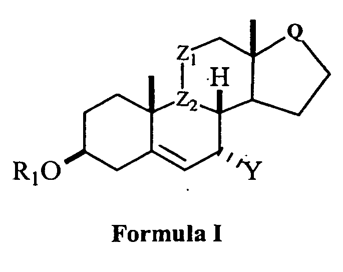 wherein
R₁ is H or -COR₂;
R₂ is C₁-C₆ alkyl or C₁-C₆ alkoxy;
Z₁ is CH₂, or 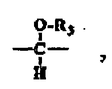 wherein OR₃ is in the a configuration;
R₃ is H or -COR₂ ;
Z₂ is -CH-; or
Z₁ and Z₂ may be taken together to form a carbon-carbon double bond;
Q is 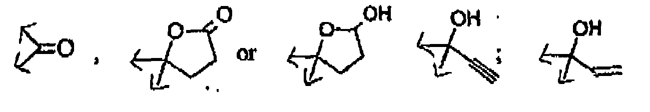
Y is -CN, -CH₂-CH=CH₂, 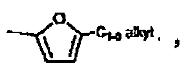 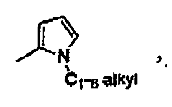 CHR₄C(O)Ar, CHR₄C(O)C₁₋₆alkyl, CHR₄C(O)XAr, or CHR₄ C(O)XC₁₋₆ alkyl;
   where
   R₄ =OC₁₋₆ alkyl or aryl
   X=O or S
comprised of reacting a steroid intermediate of Formula II; 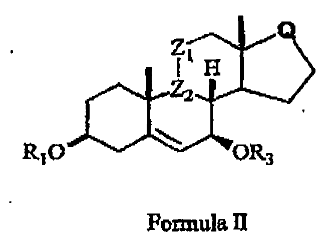 wherein R₁ and R₃, Z₁, Z₂, R₂ and Q are as for Formula I;
with a nucleophilic reagent in the presence of a Lewis acid catalyst.

In another embodiment of the invention, there is provided a compound of Formula I wherein:
wherein
R₁ is H or -COR₂;
R₂ is C₁-C₆ alkyl or C₁-C₆ alkoxy;
Z₁ is CH₂, or 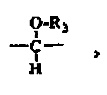 wherein OR₃ is in the a configuration;
R₃ is H or -COR₂;
Z₂ is -CH-; or
Z₁ and Z₂ may be taken together to form a carbon-carbon double bond;
Q is 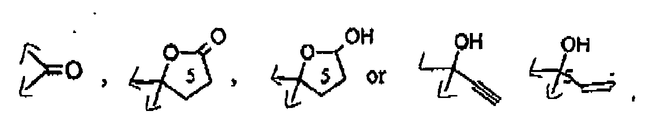
Y is -CN, -CH₂-CH=CH₂, 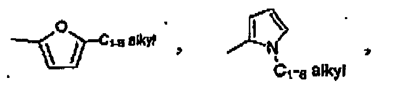 CHR₄C(O)Ar, CHR₄C(O)C₁₋₆alkyl, CHR₄C(O)XAr, or CHR₄ C(O)XC_{1-6akyl};
   where R₄ = O C_{1-6alkyl} or aryl
   X = O or S.

In another embodiment of the invention, the process further comprises steps of:
a) reacting a keto steroid of Formula I 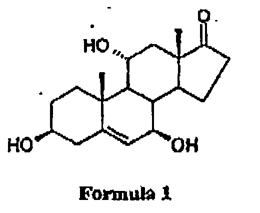 with a C₁-C₆ alkylchloroformate or benzyl chloroformate or an alkoxycarbonylbenztriazole in the presence of a tertiary organic base to give a tricarbonate of Formula 2 wherein R is C₁-C₆ alkyl or benzyl; 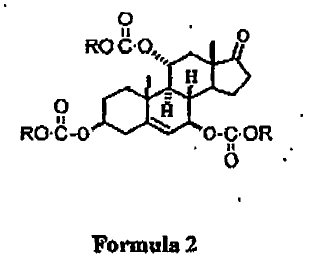
b) reacting a tri-acyl compound of Formula 2 with a 2-C₁₋₆ -alkylfuran in the presence of a Lewis acid catalyst to give a diacylester compound of Formula 3; 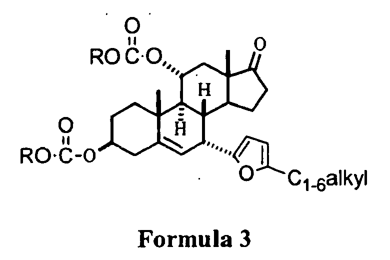
c) hydrolyzing the diacylester compound of Formula 3 in the presence of a base to give a dihydroxy ester of Formula 4; 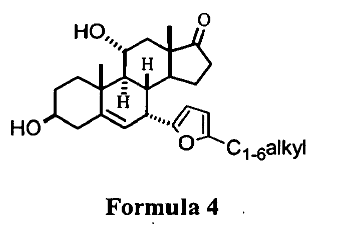
d) reacting a compound of Formula **4** with acetylene in the presence of a strong base to give an acetylenic compound of Formula 5; 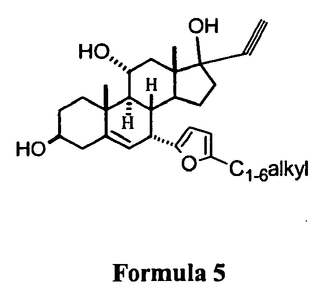
e) reacting an acetylenic compound of Formula XVII with carbon monoxide in the presence of a rhodium catalyst ligand to give a lactol of Formula **6**; 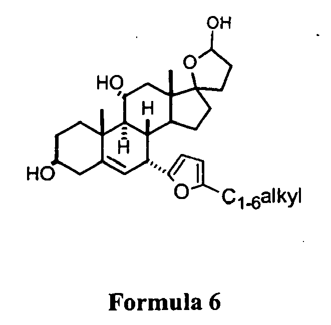
f) oxidation of a lactol of Formula 6 to give a lactone of Formula **6a**; 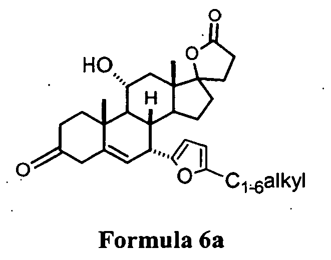
g) isomerizing the 4,5-double bond of **6a** to give a lactone of Formula **7** 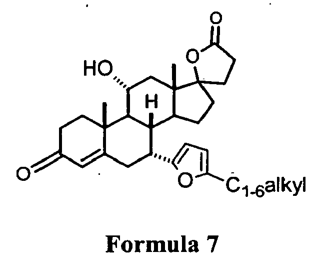
h) bromination, ozonizing, oxidizing and esterifying a compound of Formula **7** to give an ester of Formula **8**; 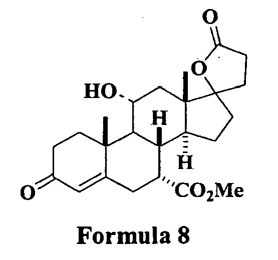
i) dehydration of a compound of Formula **8** to give an intermediate of Formula **9**; 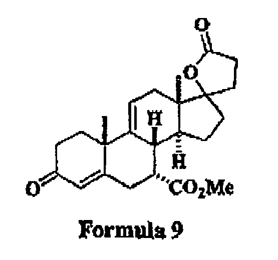
j) oxidizing a dieneone of Formula 9 whereby Eplerenone (Formula **10**) is obtained.

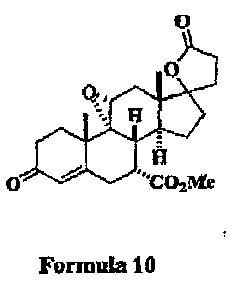

In another embodiment of the invention there is provided a compound of Formula **6a**.

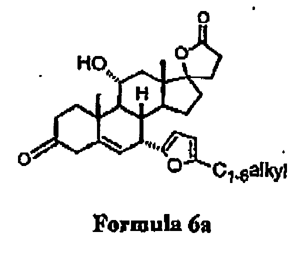

There is also provided a process for preparing 7-substituted steroid compounds of Formula I, further comprising the steps of:
a) Reacting a compound of Formula 1 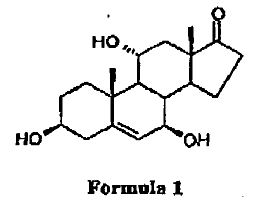 with acetylene to give a compound of Formula 11; 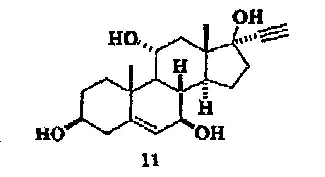
b) acylating a compound of Formula 11 to give a compound of Formula 12; 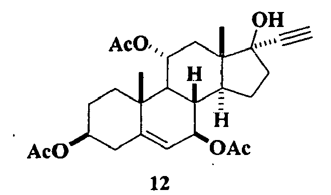
c) hydroformylating a compound of Formula 12 to give a compound of Formula 13; 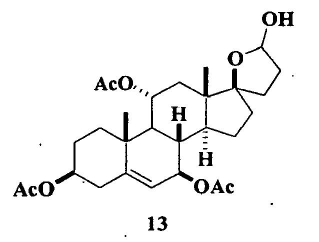
d) oxidizing a compound of Formula 13 to give a compound of Formula 14; 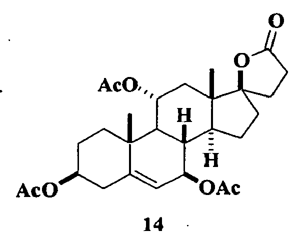
e) contacting a compound of Formula 14 with a 2-alkylfuran in the presence of a Lewis acid to give a compound of Formula 15; 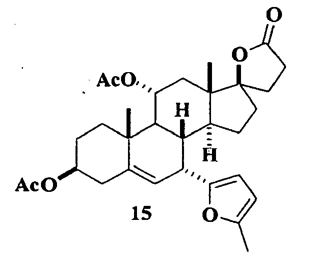
f) hydrolysing a compound of Formula 15 to give a compound of Formula 16; 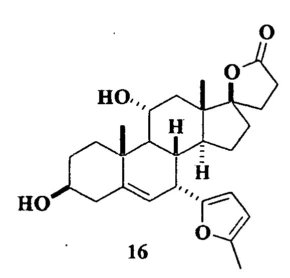
g) oxidizing a compound of Formula 16 to give a compound of Formula 17; 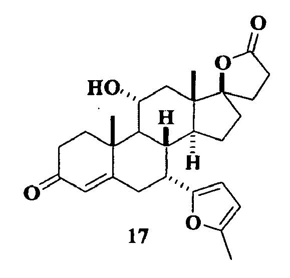
h) converting the furan ring of a compound of Formula 17 to a methoxycarbonyl compound of Formula 18; 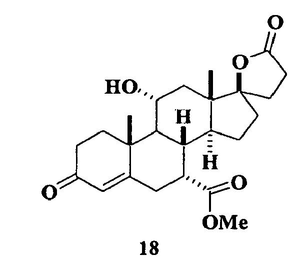
i) converting a compound of Formula 18 to a sulfonate ester of Formula 19; 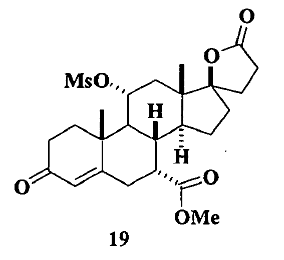
j) eliminating the sulfonate ester of Formula 19 to give a compound of Formula 9; 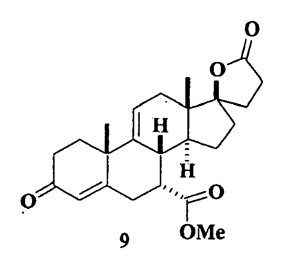
k) oxidizing a compound of Formula 9 to give a compound of Formula 10, eplerenone. 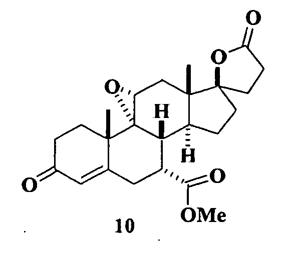 optionally further comprising silylation of a compound of Formula 1 prior to reaction with acetylene to give a silylated intermediate and removing said silyl groups during isolation of a compound of Formula 11.

### Definitions

In the detailed description, the following definitions are used. The term "alkyl," by itself or as part of another substituent, means, unless otherwise stated, a straight or branched chain, or cyclic hydrocarbon radical, or combination thereof. Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)ethyl, cyclopropylmethyl, homologs and isomers of, for example, n-pentyl, n-hexyl, n-heptyl, n-octyl, and the like.

The term "aryl," (Ar) employed alone or in combination with other terms (e.g., aryloxy, arylthioxy, aralkyl) means, unless otherwise stated, an aromatic substituent which can be a single ring or multiple rings (up to three rings) which are fused together or linked covalently.

The term nucleophilic reagent means electron rich reagents that tend to attack the nucleus of carbon as described in Morrison, R.T., et.al., *Organic Chemistry,* sixth edition, Prentice Hall pub., 1992, p. 172.

The term Lewis acid means an electron pair acceptor as defined in McQuarrie, D.A., et.al., *General Chemistry,* third edition, W.H.Freeman and Company pub., 1991, p. 665.

### Description of the Embodiments

Suprisingly, the inventors have found that carboxy derivatives of C-7-hydroxy-C-5^{Δ6}-ene steroids of Formula II undergo the allyation reaction with a variety of nucleophilic reagents to produce the corresponding C-7 substituted steroid derivatives of Formula I as shown in Table 1. Suitable nucleophilic reagents include, but are not limited to, C1-4-trialkylsilylcyanides, C1-4-trialkylsilylenolethers, trialkylsilyloxyketenethioacetals (RCH=C(OSiR₃)SR allyltri-C1-4-alkylsilanes, allyltri-C1-4-alkylstannanes, 2-C1-4alkylfurans and 2-C1-4-alkylpyrroles in the presence of a Lewis acid catalyst. Suitable Lewis acids include, but are not limited to, transition element triflates (OTf = OSO₂CF₃ such as Sc(OTf)₃, Ce(OTf)₃, Fe(ClO₄)₂, Cu(ClO₄)₂ and Yb(OTf)₃, and Molybdenum(II) complexes such as Mo(CO)₅(OTf)₂ and [Mo(CO)₄Br₂]₂.

**Table 1**

| Steroid Substrate | Nucleophilic Reagent | Catalyst | Solvent/Temp | Product | Yield % |
|---|---|---|---|---|---|
| 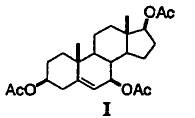 | TMSCN | Sc(OTf)₃ | Acetonitrile/RT | 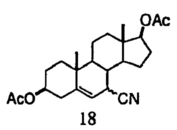 | 40 |
| 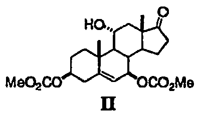 | 2-methyl furan | Sc(OTf)₃ | dichloromethane 0-15°C | 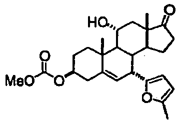 | 74 |
| 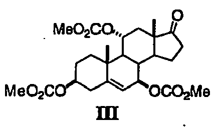 | 2-methyl furan | Sc(OTf)₃ | acetonitrile/RT | 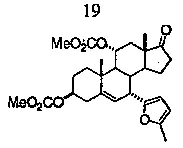 | 96 |
| 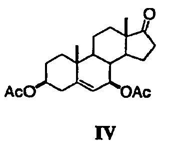 | 2-methyl furan | Sc(OTf)₃ | dichloromethane/RT | 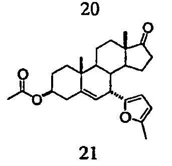 | 63 |
| | 2-methyl furan | Sc(OTf)₃ | acetonitrile/ RT | | 87 |
| | 2-methyl furan | Ce(OTf)₃ | acetonitrile/ -14°C | | 77 |
| | 2-methyl furan | Yb(OTf)₃ | acetonitrile/ RT | | 96 |
| 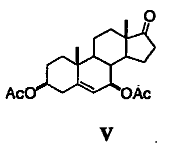 | 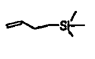 | Sc(OTf)₃ | acetonitrile/ RT | 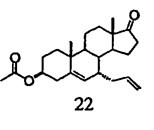 | 83 |

| | | | | | |
|---|---|---|---|---|---|
| TMSCN = trimethylsilylcyanide | | | | | |

Under similar conditions however, indole and orcinol produced yields of <10% and complex mixtures. Vinyltrimethylsilane and trimethylsilylacetylene failed to react.

### Schematic Summary

As noted above, compounds of Formula I may be used as starting materials in the synthesis of eplerenone. Schemes I and II provide a schematic flow diagram of examples of the use of a compound of Formula I, produced by the process of this invention, for the preparation of eplerenone.

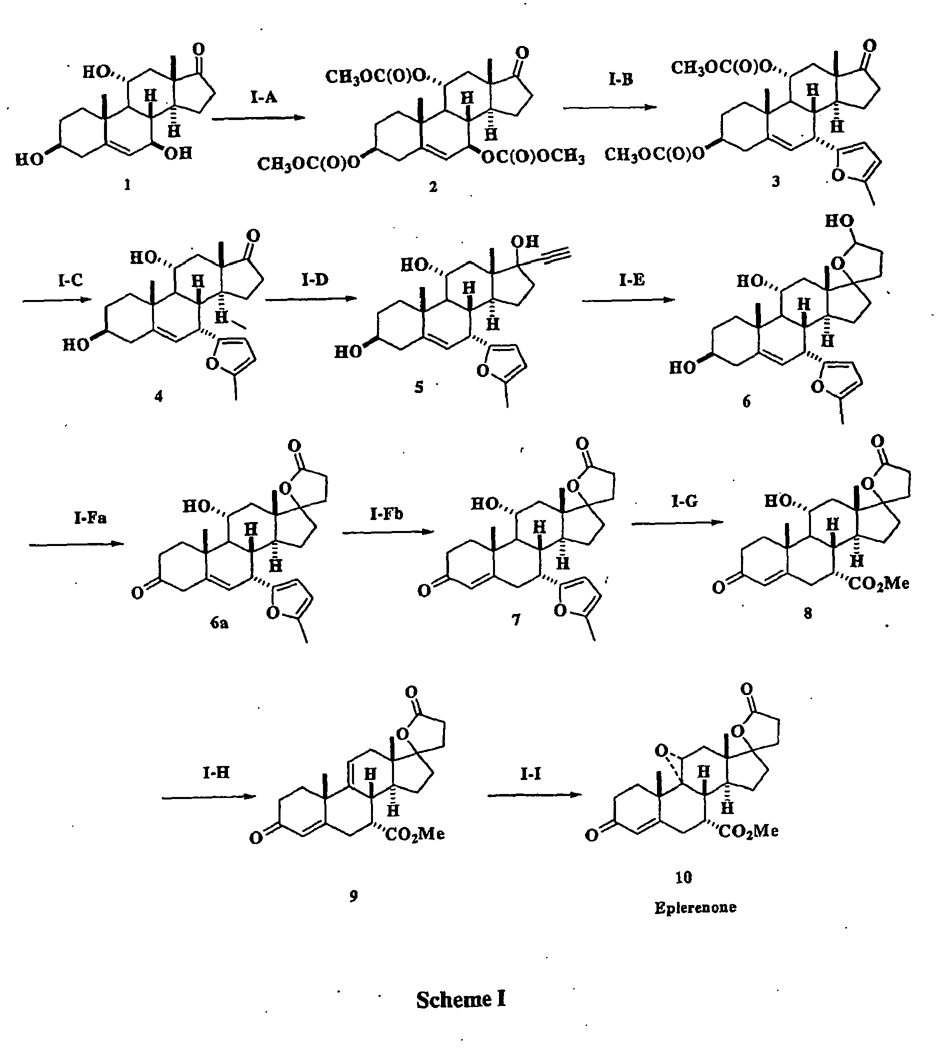

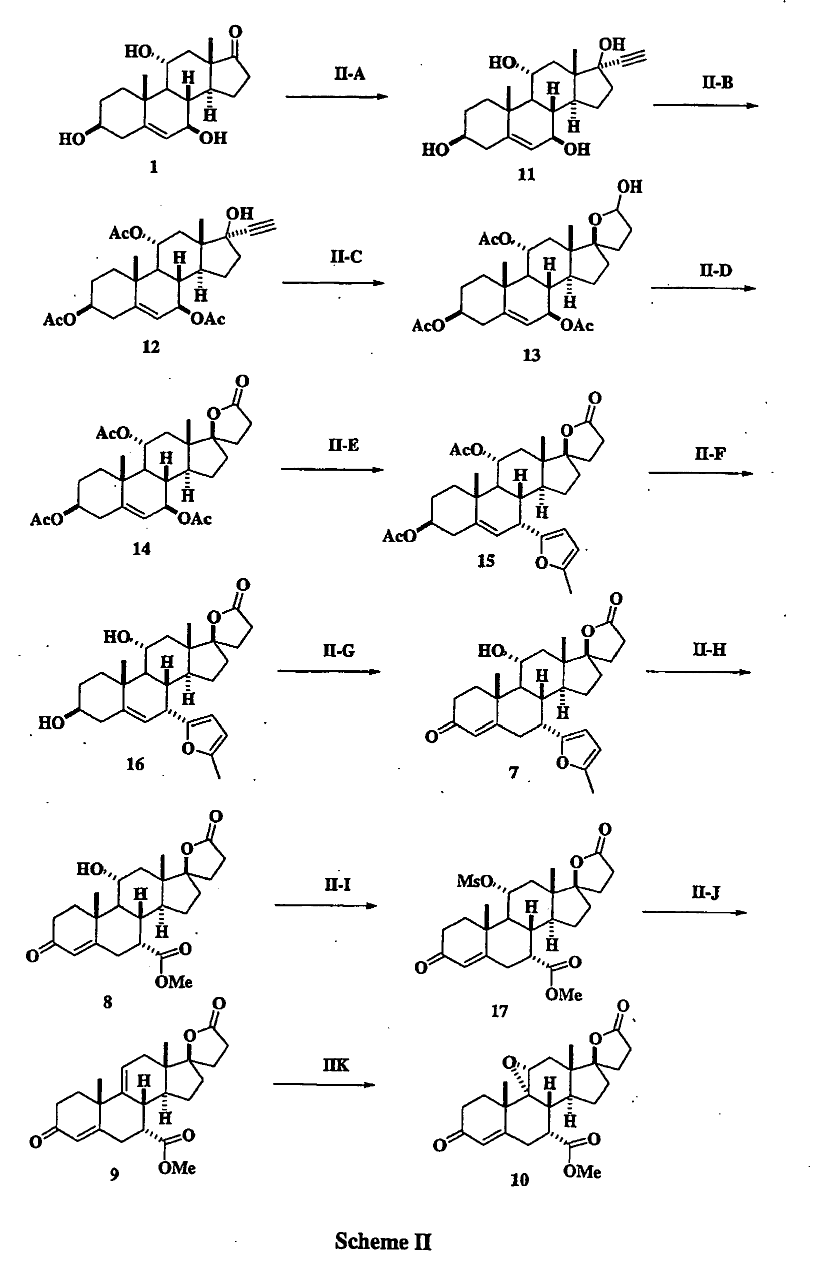

Preparation of the starting material **1** (Scheme I), (3β,7β,11α-trihydroxy-5-androsten-17-one) for Schemes I-II is obtained in one of two ways. One way is to first contact 5-anrosten-3β-ol-17-one with a submerged culture of *Diplodia gossypina* ATCC 20517 (synonym *Botryodiplodia theobromae* IFO 6469) to generate 5-androsten-3β,7β-diol-17-one (see Example 10), then to contact 5-androsten-3β,7β-diol-17-one with a submerged culture of *Aspergillus ochraceus* ATCC 18500 to generate 5-androsten-3β,7β,11α-triol-17-one 1 (Scheme I). Alternatively, one can contact 5-anrosten-3β-ol-17-one with a submerged culture of *Absidia coerulea* ATCC 6647 to generate 5-androsten-3β,7β,11α-triol-17-one **1** (Scheme I).

### Steps IA and IIB

Hydroxy intermediates **1** and **11** (Scheme II) are acylated with an acylating reagent in the presence of a tertiary organic base by procedures well known in the art to give 2 and 12. Acylating reagents include lower alkanoic anhydrides, lower alkanoic chlorides, lower alkylcarbonyl chlorides, lower alkylcarbonic anhydrides, and the like. Suitable tertiary organic bases include pyridine, 4-dimethyaminopyridine, triethylamine, diisopropylethyl amine and the like. Alternatively, preparation of mixed carbonates (RO-CO-O-) can be achieved by reaction with an alkoxycarbonyloxybenztriazole in the presence of a tertiary organic base by modification of published procedures (Harada, T., et.al., J.Carbohydrate Chem., (1995), 14, 165).

### Steps I-B and II-E

Reaction of the triacylated compounds **2** (Scheme I) and **14** (Scheme II) with a nucleophilic reagent in the presence of a Lewis acid, usually in an inert solvent such as acetonitrile or methylene chloride, gives **3** (Scheme I) and **15** (Scheme II) respectivly. Suitable nucleophilic reagents include, but are not limited to, trialkylsilylcyanides, 3-silyl substituted alkenes, enol acetates, silyl enolethers, allylstannanes, N-alkylpyrroles, N,N-dialkylanilines, silyl enol thioesters, silyl enol esters and electron rich heteroaromatics such as a 2-alkyl substituted furan. Suitable Lewis acids include, but are not limited to, transition element triflates (OTf = OSO₂CF₃) such as Sc(OTf)₃, Ce(OTf)₃, and Yb(OTf)₃, and Molybdenum(II) complexes such as Mo(CO)₅(OTf)₂ and (Mo(CO)₄Br₂]₂.

### Steps I-C and II-F

Hydrolysis of the acyl groups of 3 (Scheme I) and **15** (Scheme II) to give **4** (Scheme I) or 16 (Scheme II) is achieved using an alkaline earth hydroxide, bicarbonate or carbonate such as sodium hydroxide, potassium carbonate, sodium bicarbonate, cesium hydroxide, lithium bicarbonate and the like, using methanol as a solvent, optionally with a co-solvent.

### Steps I-D and II-A

The 17-oxo intermediates **4** (Scheme I) and **1** (Scheme I) are reacted with acetylene to provide the corresponding addition compound **5** (Scheme I) and 11 (Scheme II) according to procedures described in the literature (see for example: Schwede, W., et.al., Steroids, (1998), **63** 166; Corey, E. J. et.al., J. Amer.Chem. Soc. (1999), 121, 710-714; Schwede, W. et.al., Steroids (1998), 63(3), 166-177; Ali, H. et.al., J. Med. Chem. (1993), 36(21), 3061; Turuta, A. M. et.al., Mendeleev Commun. (1992), 47-8; Kumar, V. et.al., Tetrahedron (1991), 47(28), 5099; Page, P. C., Tetrahedron (1991), 47, 2871-8; Curts, S.W. et.al., Steroids (1991), 56, 8; Kataoka, H. et.al., Chem. Lett. (1990), 1705-8; Christiansen, R.G. et.al., J. Med. Chem. (1990), 33(8), 2094-100). Optionally, the trihydroxy compound **1** in step II-A may be trimethylsilylated without isolation before the addition of acetylene. Silylation is achieved with hexamethyldisilazane and a mild acid catalyst such as trimethylsilyl chloride or saccharin. Following the addition of acetylene, the trimethylsilyl groups are removed during work-up of the reaction with mild mineral acid, acetic acid, phosphoric acid, tetra-alkylammonium fluoride and the like.

### Step E

Formation of the lactol intermediates **6** (Scheme I) and **13** (Scheme II) is achieved by hydroformylation of 5 and 12 with carbon monoxide and hydrogen in the presence of a catalytic amount of rhodium catalyst and a rhodium coordinating ligand according to procedures described in the literature (Wuts, P.G.M., et.al., J.Org.Chem. 1989, 54, 5180; Botteghi, C., et. al., Tetrahedron, 2001, 57, 1631). The reaction is conducted at a pressure of from 14-500 psi, preferably from 100-200 psi. The ratio of hydrogen to carbon monoxide is 1/5 to 5/1, usually 1/1. Suitable rhodium catalysts include rhodium acetate, rhodium chloride and dicarbonyl acetylacetonato rhodium II. Suitable ligands include triarylphosphines, trialkyl phosphites bidentate phosphines such as xantphos, bidentate phosphites and the like.

### Steps I-Fa and II-D

Oxidation of 6 (Scheme I) to 6a (Scheme I) and 16 (Scheme II) to 7 (Scheme II) can be achieved with a variety of standard oxidizing reagents. Examples of suitable oxidizing reagents include: Iodosuccinimide/tetrabutyl ammonium iodide (Kraus, George A. Bioorganic & Medicinal Chemistry Letters (2000), 10(9), 895-897; Barrett, A.G. M., et.al., J. Org. Chem. (1989), 54(14), 3321); Jones reagent (chromic acid in acetone) (Panda, J., et.al., Tetrahedron Letters (1999), 40, 6693; Tomioka, K., et.al., J. Org. Chem. (1988), 53(17), 4094); Silver carbonate (Chow, T. J., et.al., J. Chem. Soc., Perkin Transactions 1, (1999), 1847); Pyridinium chlorochromate (Uchiyama, M., et.al., Tetrahedron Letters (2000), 41(51), 10013; Vanderiei, J. M. de L., Synthetic Communications (1998), 28(16), 3047; Kassou, M., et.al., Journal of Organic Chemistry (1997), 62, 3696; Rehnberg, N., et.al., J. Org. Chem. (1990), 55(14), 4340-9; RuO₄ / tetralkylammonium salts / tert-amine N-oxide, Jeewoo, K., et.al., Chem. Lett. (1995), (4), 299; pyridinium dichromate, Paquette, L..A., et.al., J. Am. Chem. Soc. (1995), 117(4), 1455-6); sodium hypochlorite/ tert-amine N-oxide (Waldemar, A., et.al., Chem. Rev., (2001), 101, 3499); aluminum alkoxides/acetone (Ooi, T., et.al., Synthesis (2002), 279) ; Djerassi,C. et.al., Org. React. (1951), 6, 207); triacetoxyperiodoindane (Martin, J.C., et.al., J.Amer.Chem.Soc., (1991), 113, 7277).

### Step Fb

In those instances where the oxidation of Step Fa results in the unconjugated 5-6 double bond, migration of the double bond in 6a (Scheme I)from the C₅₋₆ position to the C₄₋₅ position is accomplished by contacting 6a (Scheme I) with an organic or inorganic acid in an inert organic solvent or an aqueous mixture of solvents at a temperature of from 0°-80°C. Suitable organic acids include, but are not limited to, toluene sulfonic acid, methane sulfonic acid, benzene sulfonic acid, trifluroacetic acid, oxalic, trichloroacetic acid and the like. Suitable inorganic acids include, but are not limited to, hydrochloric acid, hydrobromic acid, phosphoric acid, perchloric acid and the like. Alternatively, the catalyst can be a tertiary organic base such as triethylamine, diazabicycloundecane (DBU) and the like or an inorganic base such as sodium hydroxide, potassium hydroxide, calcium hydroxide and the like. The double bond migration has been described (Bakshi, et.al., U.S. Patent No. 5,237,064; Pollack, et.al., J. Amer. Chem. Soc., 1987, 109, 5048; Tsubuki, et.al., J. Org. Chem., 1992, 57, 2930; Zeng, et.al.. J. Amer. Chem. Soc., 1991, 113, 3838).

### Steps I-H and II-I,J

Dehydration of 11-hydroxy intermediates **7** (Scheme I) and **18** (Scheme II) is achieved using phosphorous pentachloride as has been described (U.S. Patent 4,559,332). Alternatively, the 11-hydroxy intermediates may be converted to a sulfonyl ester, for example a methane sulfonate or a p-toluene sulfonate, followed by treatment with a base to affect the dehydration as is described in WO97/21720 and WO98/25948.

### Steps I-H and II-H

Degradation of the furan ring in 7 (Scheme I) to the methyl ester of **8** (Scheme II) is achieved by ozonolysis, oxidation and esterification as described in the examples.

### Steps I-J and II-K

Conversion of the known intermediate **9** (Scheme I) to **10** (Scheme I) (eplereneone) is described in U.S. Patent nos. 4,559,332, and 5,981,744.

### Examples

### Preparation of starting material 1, method 1.

### Step 1: Bioconversion of 5-androsten-3β-ol-17-one to 5-androsten-3β,7β-diol-17-one

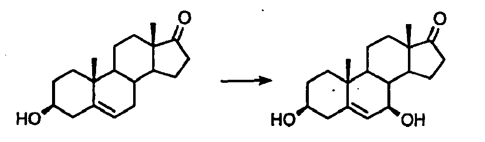

The bioconversion of 5-androsten-3β-ol-17-one to 5-androsten-3β,7β-diol-17-one is performed using a submerged culture of *Diplodia gossypina* ATCC 20571 (synonym *Botryodiplodia theobromae* IFO 6469) at a 10-L fermentation scale.

### (A) Primary-Seed Stage

Frozen vegetative cells of *Diplodia gossypina* ATCC 20571 are thawed, transferred to potato-dextrose-agar plates (PDA), and incubated at 28° for 72 hours. Single mycelial-plugs (6-7 mm diam.) are used to inoculate siliconized 500-mL stippled shakeflasks containing 100 mL primary-seed medium. Primary-seed medium consists of (per liter of RO water): dextrin, 50 g; soyflour, 35 g; cerelose, 5g; cobalt chloride hexahydrate, 2 mg; silicone defoamer (SAG 471), 0.5 mL; pre-sterilization pH 7.0-7.2, adjusted with sodium hydroxide (2N). *Diplodia gossypina* ATCC 20571 is incubated for 48 hours at 28°, using a controlled-environment incubator-shaker set at 280 r.p.m. (1" orbital stroke).

### (B) Secondary-Seed Stage

Ten-liter secondary-seed fermentations are inoculated using 1.2 mL vegetative primary-seed culture (0.012 % [v/v] inoculation rate). Secondary-seed medium contains (per liter of RO water): cerelose, 60 g; soyflour, 25 g; soybean oil, 30 mL; magnesium sulfate heptahydrate, 1 g; potassium dihydrogen phosphate, 0.74 g; polyoxyethylenesorbitan monooleate, 2 mL; silicone defoamer (SAG 471), 0.5 mL; pre-sterilization pH 3.95-4.00, adjusted with concentrated sulfuric acid. The fermentors, containing secondary-seed medium, are sterilized for 20 minutes at 121 ° using both jacket and injection steam. The agitation rate during sterilization is 200 r.p.m.. Post-sterilization, the medium pH is adjusted to 4.0 using sterile sulfuric acid (5 %). *Diplodia gossypina* ATCC 20571 is incubated at 28° using the following initial parameters: agitation, 100 r.p.m.; back pressure = 5 psig; airflow = 2.5 SLM (0.25 VVM); low DO set-point, 30 %; pH control, none. When the DO first drops to 30 %, the airflow is increased to 5 SLM (0.5 VVM). When the culture reaches low DO again, 30 % DO is maintained using agitation control. Secondary-seed cultures are harvested at approximately 60 hours post-inoculation, when the OUR is between about 10 and about 15 mM/L/h.

### (C) Steroid Bioconversion

Ten-liter steroid-bioconversion fermentations are inoculated using 500 mL vegetative secondary-seed culture (5 % [v/v] inoculation rate). Steroid-bioconversion medium is the same as secondary-seed medium. Sterilization conditions and pH adjustment are as described for secondary-seed medium. *Diplodia gossypina* ATCC 20571 is incubated at 28° using essentially the same initial parameters as those used for secondary-seed cultivation, with the exception that the low DO set-point is increased from 30 % to 50 %. When the DO first drops to 50 %, the air flow is increased from 2.5 SLM (0.25 VVM) to 5 SLM (0.5 VVM). When the culture reaches low DO again, 50 % DO is maintained using agitation control. Starting at 24 hours post-inoculation, micronized 5-androsten-3β-ol-17-one, slurried in a minimal volume of 0.2 % polyoxyethylenesorbitan monooleate, is added to the fermentation in one-hour intervals until 400 g total is added. At about 3 days post-inoculation, an additional 100 g cerelose is added to the 10-L fermentation.

Bioconversion cultures are assayed on a daily basis for 5-androsten-3β,7β-diol-17-one using TLC. One milliliter of whole beer is extracted with 10 mL methanol. Cells are separated from the aqueous-methanol mixture by centrifugation (3,000 x g for 10 minutes), and several microliters applied to a TLC plate. The TLC plate is developed in cyclohexane:ethyl acetate:methanol (90:60:15) and the product visualized by spraying the TLC with 50% sulfuric acid, followed by charring in an oven. Product is compared with authentic standard, which turns blue on spraying with 50 % sulfuric acid. Bioconversion of 5-androsten-3β-ol-17-one to 5-androsten-3β,7β-diol-17-one is complete approximately 4 days post-inoculation.

### (D) Isolation Procedure

The whole beer at harvest is centrifuged and the rich solids are recovered by centrifugation. The rich solids are extracted with 10 liters of methylene chloride and the rich extract is recovered by centrifugation. The extract is polished and concentrated to about 1-liter by distillation and the crystal slurry is cooled to -10°C. The crystals are recovered by filtration, washed with cold methylene chloride to remove color, and dried to give 227 grams of purified crystalline 5-androsten-3β,7β-diol-17-one.

### Step 2: Bioconversion of to 5-androsten-3β,7β-diol-17-one to 5-androsten-3β,7β,11α-triol-17-one.

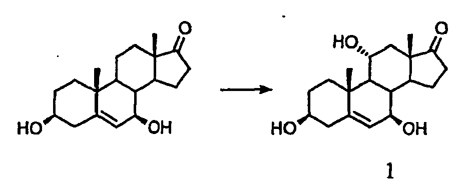

The bioconversion of 5-androsten-3β,7β-diol-17-one to 5-androsten-3β,7β,11α-triol-17-one is performed using a submerged culture of *Aspergillus ochraceus* ATCC 18500 (synonym NRRL 405) at a 10-L fermentation scale.

### (A) Primary-Seed Stage

Primary-seed cultures of *Aspergillus ochraceus* ATCC 18500 are prepared as described for *Diplodia gossypina* ATCC 20571 in Example 12.

### (B) Secondary-Seed Stage

Ten-liter secondary-seed fermentations are inoculated using 1.2 mL vegetative primary-seed culture (0.012 % [v/v] inoculation rate). Secondary-seed medium contains (per liter of RO water): cerelose, 40 g; soyflour, 25 g; soybean oil, 30 mL; magnesium sulfate heptahydrate, 1 g; potassium dihydrogen phosphate, 0.74 g; nonylphenoxypolyethoxyethanol, 0.25 mL; silicone defoamer (SAG 471), 0.5 mL; pre-sterilization pH 3.95-4.00, adjusted with concentrated sulfuric acid. The fermentors, containing secondary-seed medium, are sterilized for 20 minutes at 121 ° using both jacket and injection steam. The agitation rate during sterilization is 200 r.p.m.. Post-sterilization, the medium pH is adjusted to 4.0 using sterile sulfuric acid (5 %). *Aspergillus ochraceus* ATCC 18500 is incubated at 28° using the following initial parameters: agitation, 100 r.p.m.; back pressure = 5 psig; airflow = 2.5 SLM (0.25 VVM); low DO set-point, 50 %; pH control, none. When the DO first drops to 50 %, the airflow is increased to 5 SLM (0.5 VVM). When the culture reaches low DO again, 50 % DO is maintained using agitation control. Secondary-seed cultures are harvested between 50 to 54 hours post-inoculation, when the OUR is between about 20 and about 26 mM/L/h.

### (C) Steroid Bioconversion

Ten-liter steroid-bioconversion fermentations are inoculated using 500 mL vegetative secondary-seed culture (5 % [v/v] inoculation rate). Steroid-bioconversion medium is essentially the same as secondary-seed medium, with the exception that the nonylphenoxypolyethoxyethanol is increased from 0.25 mL/L to 2 mL/L, and pre-sterilization pH is adjusted to 2.95-3.00 with concentrated sulfuric acid. Sterilization conditions are as described for secondary-seed medium. Post-sterilization, the medium pH is adjusted to 3.0 using sterile sulfuric acid (5 %). *Aspergillus ochraceus* ATCC 18500 is incubated at 28° using essentially the same initial parameters as those used for secondary-seed cultivation, with the exception that agitation is initially set at 200 r.p.m..At about 18 hours post-inoculation, 200 g micronized 5-androsten-3β,7β-diol-17-one, slurried in a minimal volume of 0.2 % nonylphenoxypolyethoxyethanol, is added to the 10-L fermentation.

Bioconversion cultures are assayed on a daily basis for 5-androsten-3β,7β,11α-triol-17-one using TLC, as described in EXAMPLE 10. Bioconversion of 5-androsten-3β, 7β-diol-17-one to 5-androsten-3β,7β,1 1α-triol-17-one is complete approximately 4 days post-inoculation.

### (D) Isolation Procedure

The whole beer solids are recovered by centrifugation. The liquid is discarded. The rich solids are extracted with 10 liters of 80% acetone 20% water at 45°C to 50°C and the warm extract is clarified by filtration. The rich filtrate is concentrated by distillation to remove acetone generating an aqueous slurry of crude crystals. The crude crystals are recovered by filtration and the mother liquor is discarded. The water-wet crystals are triturated in 600 milliliters of methylene chloride to remove impurities, dissolved in 700 milliliters of methanol (by heating to 55°C), and then decolorized with 5 grams of Darco G-60 carbon. After filtration to remove carbon, the filtrate is concentrated to crystallize the product. The methanol is removed further by adding 300 mL of n-butyl acetate and concentrating to a thick crystal slurry. The crystals are filtered, washed with n-butyl acetate, and dried to give 158 grams of purified crystalline 5-androsten-3β,7β,11α-triol-17-one.

### Preparation of 1, Method 2: Bioconversion of 5-androsten-3β-ol-17-one to 5-androsten-3β, 7β,11α-triol-17-one.

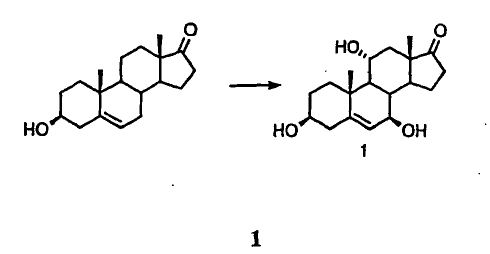

The bioconversion of 5-androsten-3β-ol-17-one to 5-androsten-3β,7β,11α-triol-17-one is performed using a submerged culture of *Absidia coerulea* ATCC 6647 at a 10-L fermentation scale.

### (A) Primary-Seed Stage

Primary-seed cultures of *Absidia coerulea* ATCC 6647 are prepared as described for *Diplodia gossypina* ATCC 20571 in EXAMPLE 12.

### (B) Secondary-Seed Stage

Ten-liter secondary-seed fermentations are inoculated using 1.2 mL vegetative primary-seed culture (0.012 % [v/v] inoculation rate). Secondary-seed medium contains (per liter of RO water): dextrin, 50 g; soyflour, 35 g; cerelose, 5 g; cobalt chloride hexahydrate, 2 mg; silicone defoamer (SAG 471), 0.5 mL; pre-sterilization pH 4.95-5.00, adjusted with concentrated sulfuric acid. The fermentors, containing secondary-seed medium, are sterilized for 20 minutes at 121° using both jacket and injection steam. The agitation rate during sterilization is 200 r.p.m.. Post-sterilization, the medium pH is adjusted to 5.0 using sterile sulfuric acid (5 %). *Absidia coerulea* ATCC 6647 is incubated at 28° using the following initial parameters: agitation, 100 r.p.m.; back pressure = 5 psig; airflow = 2.5 SLM (0.25 VVM); low DO set-point, 50 %; pH control, none. When the DO first drops to 30 %, the airflow is increased to 5 SLM (0.5 VVM). When the culture reaches low DO again, 30 % DO is maintained using agitation control. Secondary-seed cultures are harvested about 76 hours post-inoculation, when the OUR is between about 4 and about 7 mM/L/h.

### (C) Steroid Bioconversion

Ten-liter steroid-bioconversion fermentations are inoculated using 500 mL vegetative secondary-seed culture (5 % [v/v] inoculation rate). Steroid-bioconversion medium contains (per liter of RO water): dextrin, 50 g; soyflour, 35 g; cerelose, 20 g; silicone defoamer (SAG 471), 0.5 mL; pre-sterilization pH 2.95-3.00, adjusted with concentrated sulfuric acid. Sterilization conditions are as described for secondary-seed medium. Post-sterilization, the medium pH is adjusted to 3.0 using sterile sulfuric acid (5 %). *Absidia coerulea* ATCC 6647 is incubated at 28° using the same initial parameters as those used for secondary-seed cultivation. At about 17 hours post-inoculation, 200 g micronized 5-androsten-3β-ol-17-one, slurried in a minimal volume of 0.2 % octylphenoxypolyethoxyethanol, is added to the 10-L fermentation.

Bioconversion cultures are assayed on a daily basis for 5-androsten-3β,7β,11α-triol-17-one using TLC, as described in EXAMPLE 1. Bioconversion of 5-androsten-3β-ol-17-one to 5-androsten-3β,7β,11α-triol-17-one is complete approximately 6-7 days post-inoculation.

### (D) Isolation Procedure

The whole beer solids are recovered by centrifugation. The liquid is discarded. The rich solids are extracted using 10 liters of 85% acetone 15% water at 45°C to 50°C and the warm extract is clarified by filtration. The rich filtrate is concentrated by distillation to remove acetone generating an aqueous slurry of crude crystals. The crystal slurry is filtered and the mother liquor is discarded. The water-wet crystals are triturated in 600 milliliters of methylene chloride to remove impurities, dissolved in 700 milliliters of methanol (by heating to 55°C), and then decolorized with 5 grams of Darco G-60 carbon. After filtration to remove carbon, the filtrate is concentrated to crystallize the product. The methanol is removed further by adding 300 mL of n-butyl acetate and concentrating to a thick crystal slurry. The crystals are filtered, washed with n-butyl acetate, and dried to give 75.5 grams of crude crystalline 5-androsten-3β,7β,11α-triol-17-one.

The crude crystals are triturated in 600 milliliters of methylene chloride to remove additional impurities, dissolved in 700 milliliters of methanol (by heating to 55°C), and then decolorized with 5 grams of Darco G-60 carbon. After filtration to remove carbon, the filtrate is concentrated to crystallize the product. The methanol is removed further by adding 300 mL of n-butyl acetate and concentrating to a thick crystal slurry. The crystals are filtered, washed with n-butyl acetate, and dried to give 42.1 grams of purified crystalline 5-androsten-3β,7β,11α-triol-17-one.

### Example 1: Formation of tricarbonate 2.

To a 3N 250 ml RBF was charged triol 1 (Scheme 1) (10.00g, 31 mmol) dissolved in pyridine (100 ml). To this solution was added triethylamine (31 ml, 218 mmol), carbomethoxybenztriazole (24.2 g, 125 mmol), and 4-N,N-dimethylaminopyridine (1.2 g, 9.4 mmol). The slurry was stirred for 2 hours at which time everything dissolved. Additional carbomethoxybenztriazole (12 g, 62 mmol) and triethylamine (10 ml, 73 mmol) were added. Once solids dissolved the reaction was complete.

Water (300 ml) was slowly added and the mixture cooled in an ice bath. The precipitate was filtered and washed with 10% HCl (2 x 35 ml) and hexane (3 x 50 ml) and dried in vacuum oven for 24 hours to give the title compound 2 (Scheme I). ¹³C NMR (CDCl₃) δ 217.78, 155.60, 155.23, 154.88, 144.48, 122.35, 78.58, 76.81, 75.39, 55.29, 54.93, 51.09, 49.47, 47.79, 38.48, 37.89, 36.19, 36.08, 27.96, 23.58, 19.07, 14.40.

### Example 2: Formation of furan 3. (Scheme I)

A solution of the tricarbonate 2 (1.0g, 2.02 mmol) in 7 mL of acetonitrile at rt was treated with 2-methylfuran (0.2 mL, 2.22 mmol) and 0.298g of Sc(OTf)₃ for 1 h. TLC (30% EtOAc/Hex) shows the reaction to be complete. Chromatography on silica gel with 25% EtOAc/Hex affords 0.92g (96% yield) of the furan 3. ¹³C NMR (CDCl₃) δ 217.88,171.08, 155.34,154.93,152.38, 151.49, 140.72, 123.98, 110.56, 106.45, 77.50, 75.89, 60.51, 54.98, 54.71, 47.45, 46.57, 38.73, 37.66, 36.21, 35.91, 27.96, 22.22, 19.14, 13.98, 13.77.

### Example 3: Formation of diol 4. (Scheme I)

A solution of dicarbonate 3 (1.0 g) in 10 mL of MeOH is treated with 500 mg of K₂CO₃ and warmed to 40°C. The mixture is allowed to stir until TLC shows the reaction complete. When complete the slurry is poured into water and the product isolated with EtOAc. Concentration of the organic gives the diol 4 as a viscous oil. ¹H NMR (CDCl₃) δ 5.7 (s, H), 5.45 (d, J = 5.7 Hz, 1H), 3.45 (m, 1H), 3.29 (t, J = 5.1 Hz, 1H), 2.09 (s, 3 H), 1.1 (s, 3 H); 0.75 (s, 3 H).

### Example 4: Formation of alkyne 5. (Scheme I)

A solution of 2.8g (25.0 mmol) of *t*-BuOK in 50 mL of THF at -10°C was sparged with acetylene for 30 min. A solution of the ketone 11 in 10 mL of THF was then slowly added while continuing the acetylene sparge. The mixture was stirred at -10°C for 1 h and then 2.0 mL of acetic acid in 10 mL of water was added. The product was isolated by EtOAc extraction after pouring the mixture into water. Toluene was used to remove the acetic acid by azeotrope. The NMR spectra demonstrate the presence of small amounts of acetic acid and toluene; Yield 2.25 g.¹³C NMR (CDCl₃) δ 153.77, 151.21, 142.66, 122.83, 110.12, 106.1, 87.3, 79.42, 74.03, 72.29, 69.48, 50.61, 47.49, 45.70, 43.64, 42.87, 39.38, 39.06, 38.29, 37.68, 31.84, 23.7, 21.26, 19.27, 14.19, 13.9.

### Example 5: Formation of lactol 6. (Scheme I)

A solution of 1.55 g of the alkyne **5** (Scheme I), 27mg of Rh₂(OAc)₂ and 92 mg of Ph₃P in 20 mL of EtOAc was pressurized to 100 psi with CO and 100 psi with H₂ and heated to 80°C over night. The mixture was concentrated and chromatographed on silica with 90% EtOAc/ Hex to give 2 fractions. Faction 1, was shown by NMR to be recovered starting material. Fraction 2 was the desired lactol. CMR shows signals for the lactol mixture at 94.8 and 94.5 ppm.

### Example 6: Formation of 5,6-enone 6a. (Scheme I)

A mixture of 2.0 g of the lactol, 50 mg of KBr, 12 mg of TEMPO, 800 mg of NaHCO₃ in 20 mL of CH₂Cl₂ and 5 mL of water is cooled to 5°C. This mixture is then slowly treated with 8 mL of 1.1 M NaOCl while keeping the temperature below 6°C. After the addition the mixture is stirred an addition 30 min and then the product is isolated with CH₂Cl₂ to afford 6a (Scheme I). ¹³C NMR (CDCl₃) δ 209.82,176.37, 153.19, 151.78,143.34, 128.15, 121.41, 110.62, 106.53, 94.35, 72.0, 55.39, 50.44, 47.99, 44.41, 42.26, 39.03, 38.63, 37.1, 35.67, 31.9, 29.19, 23.39, 18.33,15.69, 14.07.

### Example 7: Formation of 4,5-eneone 7 (Scheme I) with acid.

A mixture of 5,6-eneone **6a** (Scheme I) (500 mg) and oxalic acid (200 mg) in ethanol (10 ml) is heated at 40°C for 3 hr. The ethanol is removed under reduced pressure and the residue dissolved in ethyl acetate (50 ml), the organic solution washed with water (2X 50 ml), dried over sodium sulfate and concentrated. The residue is purified by column chromatography on silica gel to give the 4,5-enone 7 (Scheme I).

### Example 8: Formation of 4,5-eneone 7 with base.

A mixture of 5,6-eneone **6a** (500 mg) and DBU (200 mg) in tetrahydrofuran (5 ml) is heated at reflux for 3. hr. then coolec, diluted with ammonium chloride solution and extractedwith ethyl acetate. The extract is dried over sodium sulfate and concentrated. The residue is purified by column chromatography on silica gel to give the 4,5-enone **7**.

### Example 9: Formation of dieneone 9 (Scheme I).

A solution of 1.2 g of the alcohol 7 is dissolved in 10 mL of THF and cooled to -33°C. PCl₅ (950 mg) is then added all at once. The solution is stirred for 3 h at -33°C and then quenched by adding water. The product is isolated with EtOAc to give the diene **9** (Scheme I). It is purified by silica gel chromatography with EtOAc/Hexane.

### Example 10: Formation of methyl esters from furan substituents.

### Method A

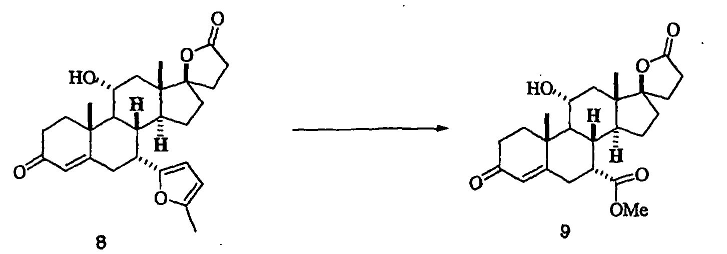

A solution of furan derivative **8** (Scheme I) (1.0 g, 2.280 mmoles) in 100 ml methylene chloride was cooled to -79°C. A stream of O₃/O₂ was passed through the solution for 10 min., then the mixture was warmed to room temp. and concentrated to a solid residue, which was taken up in 50 ml 1/1 methanol/methylene chloride, treated with 1.0 ml of pyridine, and stirred at room temp. for 18 hours. The solution was then cooled to -80°C. A stream of O₃/O₂ was then passed through the solution for 4 minutes. The mixture was then diluted with 100 ml ethyl acetate and extracted with 70 ml aq. sodium bicarbonate. The aqueous phase was acidified with aq. hydrochloric acid to pH 0.5, then extracted with methylene chloride and concentrated to a foam (weight: 250 mg). The foam was dissolved in toluene/methanol, treated with trimethylsilyldiazomethane (0.5 ml of 2.0 M solution in hexane, 1.0 mmoles) at room temp., then the solution was concentrated to give ester 9 as an oil.

### Method B

### Step 1) 5α,17β-Dihydroxypregn-9(11)-ene-3-one, 7α,21-dicarboxylic acid; bis-γ-lactone 8a (Scheme I).

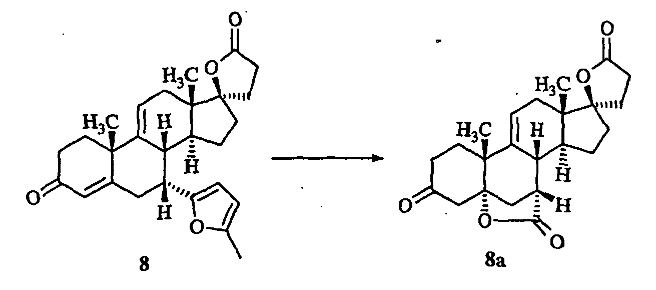

A mixture of 17β-hydroxy-7α-(5'-methyl-2'-furyl)-pregna-4,9(11)-dien-3-one-21-carboxylic acid, γ-lactone (100 g, 0.23778 moles) and potassium acetate (50.0 g, 0.5094 moles, 2.14 equivalents) in acetone (500 ml) and water (150 ml) is cooled to - 10° and treated with a slurry of dibromantin (34.0 g, 0.1189 moles, 0.50 molar equivalents) in water (100 ml) until a rise in the redox potential occurred. At this point, LC analysis indicated complete conversion into enedione (III-cis). The reaction mixture containing the enedione (III-*cis*) is then quenched with isobutyl vinyl ether (1.0 ml, 0.768 g, 7.668 mmoles, 0.032 equivalents), concentrated to a thick slurry, diluted with methylene chloride (200 ml), and treated with 20° concentrated hydrochloric acid (50.0 ml, 0.50 moles, 2.10 equivalents). The mixture is stirred at 20-25° for 2 hrs., at which time LC analysis indicated complete conversion to enedione (III-trans). The organic phase containing the enedione (III-*trans*) is separated, diluted with methylene chloride (80 ml) and methanol (300 ml), and cooled to -48°. A stream of O₃/O₂ is bubbled through this mixture until LC analysis indicated complete disappearance of the enedione (III-*trans*), then the mixture is quenched with dimethylsulfide (30.0 ml, 25.38 g, 0.4085 moles, 1.72 equivalents), stirred at -20° for 16 hrs., concentrated to a volume of -300 ml, diluted with methanol (350 ml), concentrated to a volume of about 300 ml, diluted with isopropanol (40 ml) and methanol (80 ml), then treated with a warm (55-60°) solution of potassium bicarbonate (120 g, 1.1986 moles, 5.04 equivalents) in water (240 ml). This slurry is cooled to 5-10°, then hydrogen peroxide (50%, 66.0 g, containing 33.0 g (0.9703 moles, 4.08 equivalents) hydrogen peroxide) is added over 3 hrs. The mixture is stirred for four hrs. and quenched with dimethylsulfide (40 ml, 33.84 g, 0.5447 moles, 2.29 equivalents). After stirring at 20-25° for 23 hrs., the mixture is diluted with methylene chloride (100 ml) and water (80 ml), and acidified to pH = 3.0 with concentrated hydrochloric acid. The two-phase mixture is heated to 36°, then the phases are separated and the aqueous phase extracted with methylene chloride (100 ml). The organic phases are combined, washed with water (75 ml), and the aqueous phase is back-extracted with methylene chloride (25 ml). The organic phases are combined, concentrated to a volume of 150 ml, then treated with benzenesulfonic acid (1.0 g of 90% pure material, containing 0.90 g (5.690 mmoles, 0.0239 equivalents) benzenesulfonic acid) and acetone (50 ml). The mixture is then concentrated atmospherically to a volume of 160 ml, then diluted with acetone (250 ml), concentrated to a volume of 200 ml, cooled to 12°, and filtered. The filter cake is washed with cold acetone (2 x 25 ml) and dried by nitrogen stream to give the title compound, CMR (100 MHz, CDCl₃) 206.08, 176.47, 175.41, 139.63, 124.00, 94.89, 90.97, 47.08, 43.90, 42.36, 41.58, 41.07, 38.93, 36.97, 35.16, 33.01, 32.42, 32.42, 31.35, 29.10, 23.08, 22.98 and 14.23 δ; NMR (400 MHz, CDCl₃) 0.94, 1.40, 1.4-2.8 and 5.70; MS (CI, NH₃) m/e = 385 (P + H, 100%).

### Step 2) 17β-Hydroxy-7α-carbomethoxypregna-4,9(11)-dien-3-one-21-carboxylic acid, γ-lactone 9 (Scheme I).

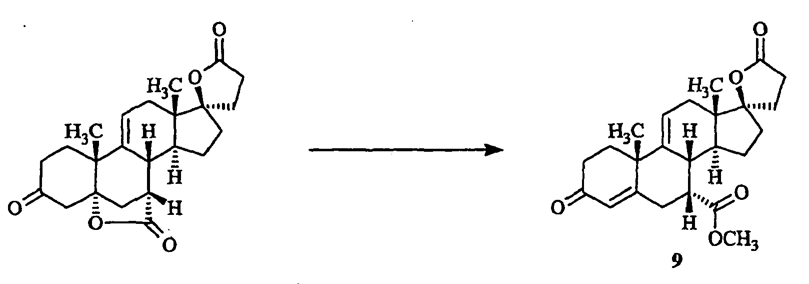

A mixture of 5α,17β-dihydroxypregn-9(11)-ene-3-one, 7α,21-dicarboxylic acid, bis-γ-lactone (50.0 g, 0.13005 moles) and potassium bicarbonate (16.92 g, 0.1690 moles, 1.30 equivalents) in acetone (200 ml) and water (100 ml) is stirred at 45° for 2 hrs., at which time conversion of the 5,7-lactone (VII) into the carboxylic acid (VI) is complete by LC. The resulting mixture is then treated with dimethylsulfate (22.92 g, 0.1817 moles, 1.40 equivalents), stirred at 45° for 3 hrs., then treated with a solution of potassium bicarbonate (1.3 g, 0.0130 moles, 0.100 equivalents) in water (10 ml) followed by neat triethylamine (1.81 ml, 1.314 g, 0.0130 moles, 0.100 equivalents). The mixture is stirred at 45° for 1 hr., quenched with concentrated hydrochloric acid (1.92 ml, 2.304 g, containing 0.852 g (0.0234 moles, 0.180 equivalents) hydrochloric acid), cooled to 0°, concentrated under reduced pressure to a volume of 150 ml (pot temperature 13°), then filtered and the filter cake is washed with water (2 x 25 ml) and dried to give the title compound 9 (Scheme I).

### Example 11: Formation of eplerenone.

Dieneone 9 (Scheme I) is oxidized as described in U.S. Pat. Nos. 4,559,332, and 5,981,744, and WO97/21720 and WO98/25948 to give eplerenone.

### Example 12: Allylation of trimethylsilylcyanide with I.

A solution of the triacetate **I** (Table 1) (1.0 g, 2.24 mmol) in 10 mL of CH₂Cl₂ was cooled to 14°C and treated with 0.6 mL of TMSCN and 100 mg of Sc(OTf)₃. The mixture was stirred for 5 h and the extracted with ethyl acetate. During concentration of the extract crystals precipitated from solution. These were filtered and dried to afford the nitrile **18** as a mixture of isomers. ¹³C NMR (CDCl₃, as the mixture) δ 147.31, 146.0, 131.68, 129.39, 128.12, 119.23, 115.47, 115.04, 62.74, 82.50, 51.13, 49.0, 47.72, 44.38, 43.67, 43.05, 37.32, 37.04, 36.32, 33.58, 32.09, 32.0, 27.92, 27.79, 26.75, 23.68, 23.32, 20.45, 19.13, 18.26, 12.30.

### Example 13: Allylation of allyltrimethylsilane with V

A solution of the triacetate **V** (Table 1) and allyltrimethylsilane in acetonitrile is treated at room temperature with Sc(OTf)₃. After 1 hour water is slowly added to precipitate the product. Filtration and drying affords the allyl derivative **19**. ¹³C NMR (CDCl₃) δ 221.05, 170.89, 193.87, 137.62, 127.15, 116.36, 74.26, 47.81, 46.29, 38.61, 37.49, 36.23, 35.61, 35.31, 31.57, 28.04, 22.61,20.56,19.64,13.48.

### Example 14: Addition of acetylene to 17-oxo intermediates.

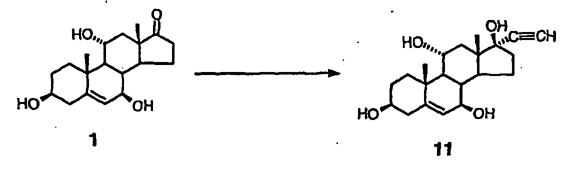

### Step 1:

Hexamethyldisilazane (HMDS) (100 ml) is added to a stirred slurry of 50.0 g Triol **1** in 400 ml methylene chloride. Saccharin (0.57 g) is added and the mixture is heated under reflux for 3 hours during which time the slurry will gradually dissolve to a clear, amber solution. Water (5 ml) is added to quench any excess HMDS. After 5 minutes at reflux the mixture is filtered through a CH₂Cl₂ wet layer of 32.6 g magnesol on a 350 ml coarse frit filter funnel. The filtrate should be clear and almost colorless. The filter cake is washed with 2 x 100 ml CH₂Cl₂. The combined filtrates are concentrated under reduced pressure and residual methylene chloride is removed by evaporation with 2 x 500 ml portions of tetrahydrofuran (THF), concentrating to dryness after each addition to give a white solid.

### Step 2:

A suspension of potassium t-butoxide (42.0 g) in 500 ml THF is cooled to-9° ± 5°C with an ice/methanol bath. Acetylene is bubbled into the mixture just under the surface with moderate stirring at for at least 1 hour. The silylated steroid intermediate from above in THF (400 ml) is added over 30 min while maintaining a reaction temperature of 0° ± 5°C. After the addition, the mixture is stirred for a further hour at 5° ± 5°C. Water (100 ml) is added slowly allowing the reaction mixture to warm up to 15° ± 5°C. 125 ml of 10 % HCl is slowly added to reduce the pH to 2.5 to 3. The mixture is stirred at pH 2.5 to 3, adding small amounts of 5 % HCl as needed to maintain a pH of 2.5 to 3, for 1 to 2 hours at 20° ± 5°C. When the hydrolysis is complete, half saturated NaHCO₃ solution is added to raise the pH to 5.5 to 6. The mixture is diluted with ethyl acetate (500 ml) and the phases separated. The aqueous phase is extracted with ethyl acetate and the combined ethyl acetate phases are washed with water, brine, dried over magnesium sulfate and concentrated to give the addition product **2.**). ¹³C NMR (DMSO-*d₆*) δ 141.99,127.38, 89.37, 77.73, 75.24, 72.13, 70.54,67.68,54.13,49.57,47.43,43.94,42.58,40.52, 40.22, 39.01, 38.09, 31.95, 25.8, 18.58, 14.09.

### Example 15: Hydroxy acetylations:

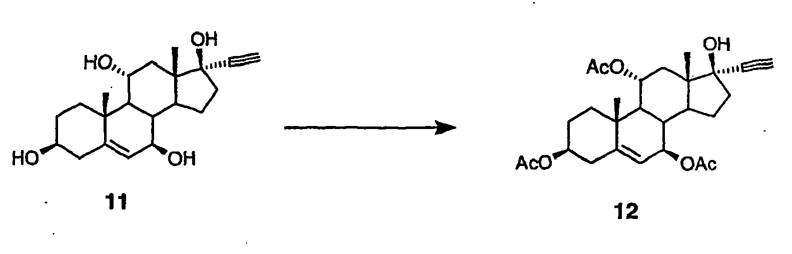

A mixture of the tetraol **11** (Scheme II) (50.00g, 144 mmol) dissolved in pyridine (150 ml) is cooled to <10°C in an ice bath. Dimethylaminopyridine (DMAP) (1.7 g, 14 mmol) is added followed by slow addition of acetic anhydride (41.4 ml, 439 mmol) at a rate to maintain the solution temperature below 10°C. Following the addition, the reaction mixture is warmed to room temperature. The mixture is diluted with ethyl acetate (75 ml) and water (50 ml), stirred for 5 minutes and the layers separated. The organic layer is washed with 10% HCl (4x25 ml) followed by H₂O (2x 50 ml), dried over MgSO₄ and concentrated. The product is recrystallized from toluene (100 ml). ¹³C NMR (CDCl₃) δ 170.68, 170.10, 143.48, 128.90, 128.10, 125.17, 122.59, 86.63, 78.21, 75.07, 74.40, 72.79, 71.47, 50.16, 48.07, 47.02, 38.76, 38.06, 37.83, 37.67, 36.92, 27.66, 24.18, 21.74, 21.44, 18.65, 13.06.

### Example 16: Hydroformylation of acetylene adducts.

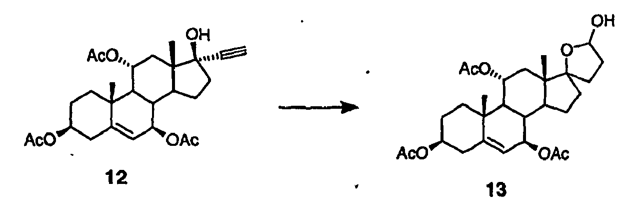

A solution of the triacetate **12** (Scheme II) (25.4 g, 54 mmol), PPh₃ (2.13 g, 8.1 mmol) and Rh₂(OAc)₄ (716 mg, 1.62 mmol) in ethyl acetate (200 ml) is heated at 80°C under a 1/1 mixture of hydrogen/carbon monoxide at a pressure of 170 psi for 12 hours. The mixture is concentrated under reduced pressure and the product 13 (Scheme II) purified by column chromatography (70/30 EtOAc/Hex and 500 g silica). The CMR spectra of this compound was complicated by ring opened and ring closed isomers and was thus not characterized fully.

### Example 17: Oxidation of lactol to lactone.

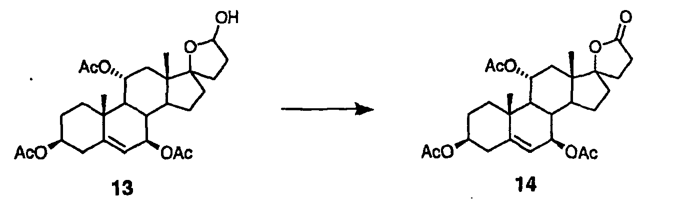

A mixture of the lactol **4 (Scheme I)** (25 g, 50 mmol), methylene chloride(250 ml), water (38 ml), 2,2,6,6-tetramethylpiperidin-1-oxyl (TEMPO) (156 mg, 1 mmol), KBr (595 mg, 5 mmol), and NaHCO₃ (5.5 g, 65 mmol) is cooled to ≤ 10°C in an ice bath. A solution of 1.1 M sodium hypochlorite (NaOCl) (50 ml, 55 mmol) is slowly added. The mixture is allowed to warm to room temperature and diluted with water (50 ml). The layers are separated and the organic layer washed with brine (2x 50ml). The organic layer is dried with MgSO₄, filtered and concentrated to afford 5 as an off white foam. ¹³C NMR (CDCl₃) δ 177.94, 172.60, 172.15, 171.58, 145.49, 124.36, 96.18, (79.22, 78.90, 78.59 CDCl₃), 76.59, 74.57, 72.63, 52.14, 49.55, 47.75, 40.00, 39.75, 39.61, 38.65, 37.47, 32.74, 30.85, 29.56, 26.01, 23.61, 23.37, 23.17, 23.11, 20.52,16.19.

### Example 18: Furanylation.

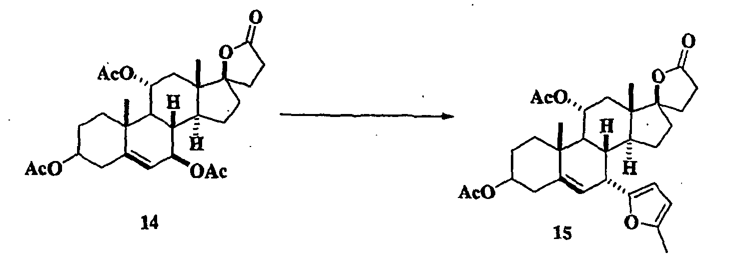

A solution of the triacetate **14** (Scheme I) (1.30g, 2.58 mmol), 2-methylfuran (0.8 mL) in 25 mL of acetonitrile at 20°C was treated with 250 mg of Sc(OTf)₃ and allowed to stir for 1 h. The product was isolated by EtOAc extraction and purified by chromatography on silica gel with 40% EtOAc/Hex to afford 1.0 g (74% yield) of the furan 15. ¹³C NMR (CDCl₃) δ 176.27, 170.45, 169.85, 152.53, 150.96, 140.60, 123.45, 110.05, 106.01, 94.95, 73.39, 71.37, 46.50, 45.40, 44.60, 38.55, 38.37, 38.06, 37.78, 37.74, 36.89, 35.41, 31.81, 30.72, 28.96, 28.93, 27.69, 23.07, 22.63, 21.74, 20.98, 18.80, 14.83, 14.13, 14.06, 13.62.

### Example 19: Acetate hydrolysis.

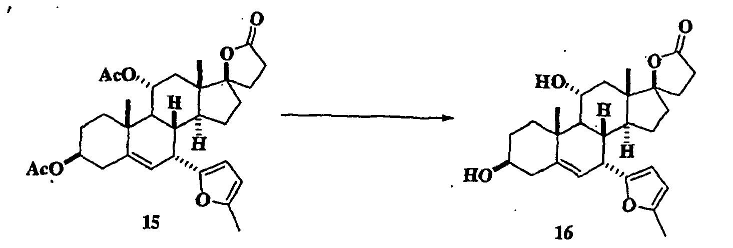

A mixture of 810 mg of the diacetate **15** (Scheme II), 112 mg of K₂CO₃ in 20 mL of methanol was stirred at rt over night. TLC indicated the reaction was not complete so an additional 100 mg of K₂CO₃ was added and stirring continued until TLC showed complete reaction. The mixture was acidified with 1M HCl and the product extracted with EtOAc. Chromatography on silica gel with 100% EtOAc affords 610 mg (89.5% yield) of the diol **16** (Scheme II). ¹³C NMR (CDCl₃) δ 176.68, 153.20, 150.79, 142.05, 122.32, 109.80, 105.94, 95.3, 71.83, 68.64, 50.13, 45.81, 44.88, 42.73, 42.62, 39.01, 38.56, 37.73, 36.81, 35.44, 31.57, 30.84, 29.06, 23.13, 18.81, 15.27, 13.64.

### Example 20: Oxidation of 16 to form 17.

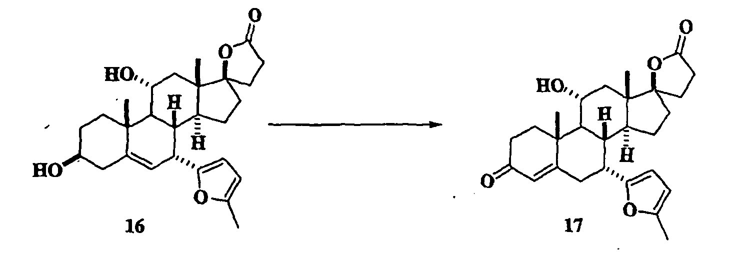

The diol **16** (Scheme II) was disolved in 2 mL of toluene and 0.1 mL of acetone and treated with 50 mg of aluminum isopropoxide and heated to 100°C After several hours there appeared to be no conversion so 0.1 mL of cyclohexanone was added and the mixture heated overnight. The product **17** (Scheme II) was isolated by silica gel chromatography using ethyl acetate as eluent. ¹³C NMR (CDCl₃) δ 199.96, 177.05, 170.32, 153.34, 150.74, 126.7, 109.14, 106.33, 95.54, 69.08, 52.50, 46.26, 46.0, 43.58, 40.13, 39.05, 38.8, 37.93, 36.92, 35.66, 34.59, 31.33, 29.47, 23.06, 18.83, 16.01, 13.90.

## Claims

1. A process for preparing 7-substituted steroid compounds of Formula I, 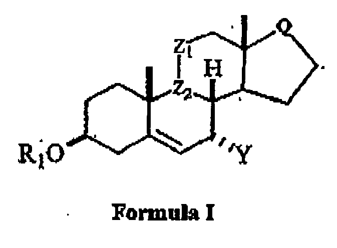 wherein
R₁ is H or -COR₂;
R₂ is C₁-C₆ alkyl or C₁-C₆ alkoxy;
Z₁ is CH₂, or 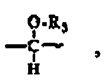 wherein OR₃ is in the α configuration;
R₃ is H or -COR₂;
Z₂ is -CH-; or
Z₁ and Z₂ may be taken together to form a carbon-carbon double bond;
Q is, 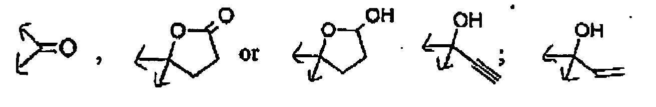
Y is - CN, -CH₂-CH=CH₂, 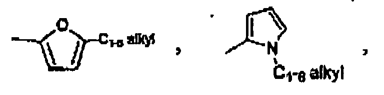 CHR₄C(O)Ar; CHR₄C(O)C₁₋₆alkyl, CHR₄C(O)XAr, or CHR₄ C(O)XC₁₋₆ alkyl;
where
R₄ = O C₁₋₆ alkyl or aryl
X = O or S
comprised of reacting a steroid intermediate of Formula II; 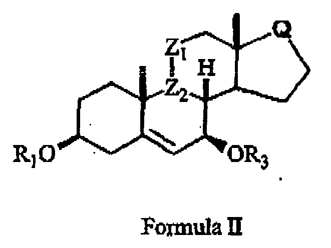 wherein R₁ and R₃ , Z₁, Z₂, R₂ and Q are as for Formula I;
with a nucleophilic reagent in the presence of a Lewis acid catalyst.

2. A compound of Formula I as described in claim 1 wherein:
wherein
R₁ is H or -COR₂;
R₂ is C₁-C₆ alkyl or C₁-C₆alkoxy;
Z₁ is CH₂, or 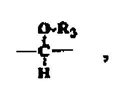 wherein OR₃ is in the a configuration;
R₃ is H or -COR₂;
Z₂ is -CH-; or
Z₁ and Z₂ may be taken together to form a carbon-carbon double bond;
Q is 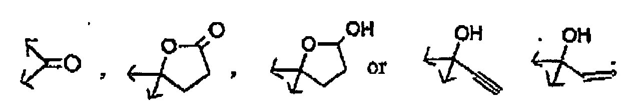
Y is -CN, -CH₂-CH=CH₂, 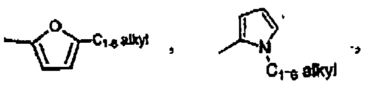 CHR₄C(O)Ar, CHR₄C(O)C₁₋₆akyl, CHR₄C(O)XAr, or CHR₄ C(O)XC₁₋₆alkyl;
Where R₄ = OC₁₋₆alkyl or aryl
X = O or S

3. A process according to Claim 1 further comprising the steps of:
a) reacting a keto steroid of Formula 1 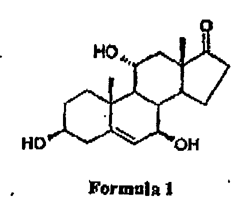 with a C₁-C₆ alkylchloroformate or benzyl chloroformate or an alkoxycarbonylbenztriazole in the presence of a tertiary organic base to give a tricarbonate of Formula 2 wherein R is C₁-C₆ alkyl or benzyl; 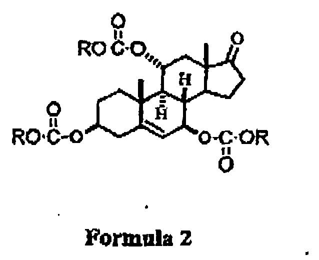
b) reacting a tri-acyl compound of Formula 2 with a 2-C₁₋₆ -alkylfuran in the presence of a Lewis acid catalyst to give a diacylester compound of Formula 3; 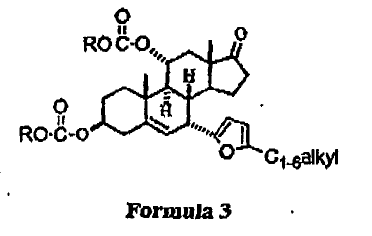
c) hydrolyzing the diacylester compound of Formula 3 in the presence of a base to give a dihydroxy ester of Formula 4; 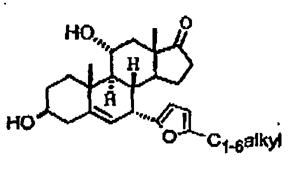 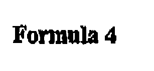
d) reacting a compound of Formula 4 with acetylene in the presence of a strong base to give an acetylenic compound of Formula 5; 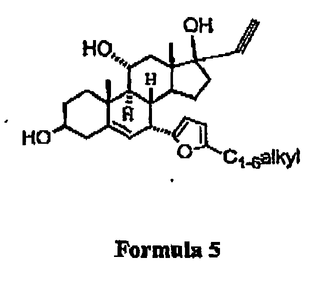
e) reacting an acetylenic compound of Formula XVII with carbon monoxide in the presence of a rhodium catalyst ligand to give a lactol of Formula 6; 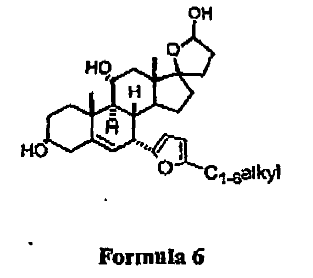
f) oxidation of a lactol of Formula 6 to give a lactone of Formula 6a; 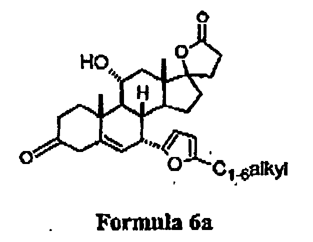
g) isomerizing the 4,5-double bond of 6a to give a lactone of Formula 7 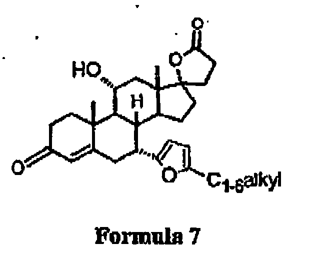
h) bromination, ozonizing, oxidizing and esterifying a compound of Formula 7 to give an ester of Formula 8; 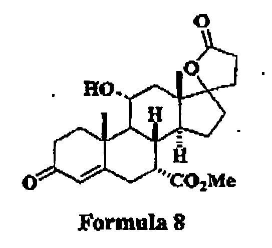
i) dehydration of a compound of Formula **8** to give an intermediate of Formula 9; 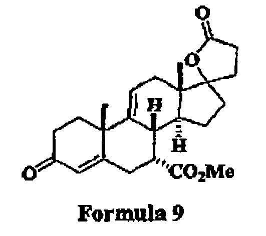
j) oxidizing a dieneone of Formula 9 whereby Eplerenone (Formula 10) is obtained.

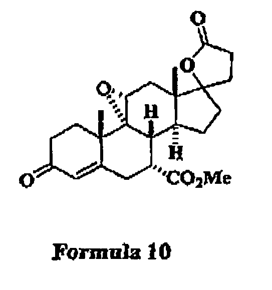

4. A compound of Formula **6a.**

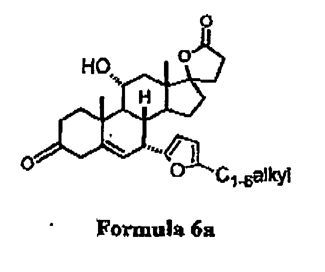

5. A process according to Claim 1 further comprising the steps of:
a) Reacting a compound of Formula 1 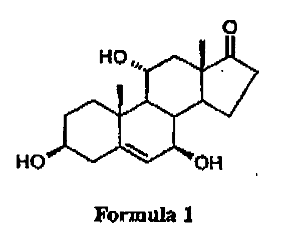 with acetylene to give a compound of Formula 11; 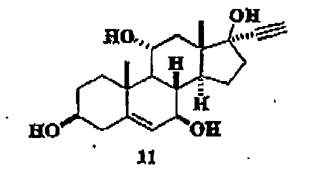
b) acylating a compound of Formula 11 to give a compound of Formula 12; 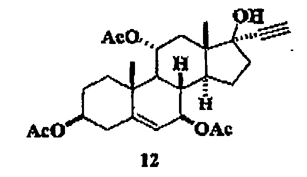
c) hydroformylating a compound of Formula 12 to give a compound of Formula 13; 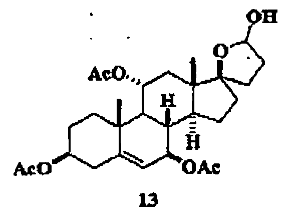
d) oxidizing a compound of Formula 13 to give a compound of Formula 14; 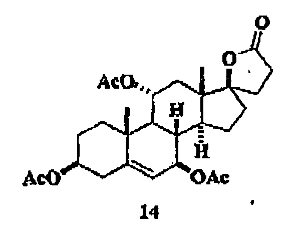
e) contacting a compound of Formula 14 with a 2-alkylfuran in the presence of a Lewis acid to give a compound of Formula 15; 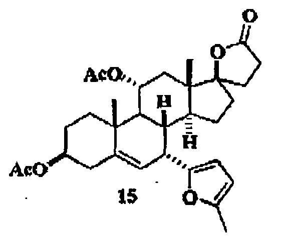
f) hydrolysing a compound of Formula 15 to give a compound of Formula 16; 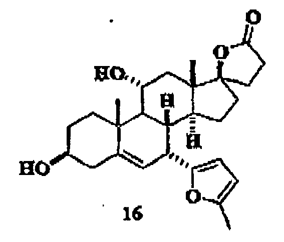
g) oxidizing a compound of Formula 16 to give a compound of Formula 17; 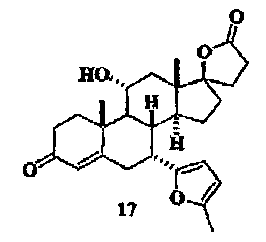
h) converting the furan ring of a compound of Formula 17 to a methoxycarbonyl compound of Formula 18; 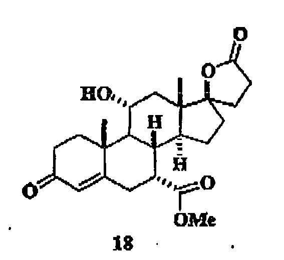
i) converting a compound of Formula 18 to a sulfonate ester of Formula 19; 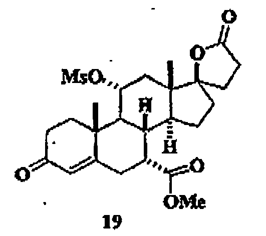
j) eliminating the sulfonate ester of Formula 19 to give a compound of Formula 9; 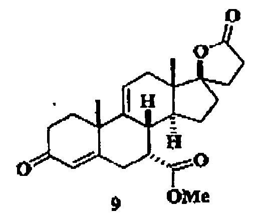
k) oxidizing a compound of Formula 9 to give a compound of Formula 10, eplerenone.

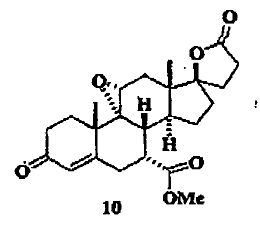

6. A process for preparing eplerenone according to Claim 5 further comprising silylation of a compound of Formula 1 prior to reaction with acetylene to give a silylated intermediate and removing said silyl groups during isolation of a compound of Formula 11.

## Patentansprüche

1. Verfahren zum Herstellen von 7-substituierten Steroidverbindungen der Formel I 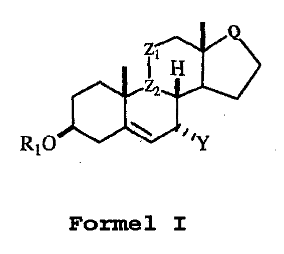 worin
R₁ H oder -COR₂ ist;
R₂ C₁-C₆Alkyl oder C₁-C₆Alkoxy ist;
Z₁ CH₂ oder 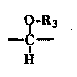 ist, worin OR₃ in der α-Konfiguration vorliegt;
R₃ H oder -COR₂ ist;
Z₂ -CH- ist; oder
Z₁ und Z₂ zusammengenommen werden können, um eine Kohlenstoff-Kohlenstoff-Doppelbindung zu bilden;
Q 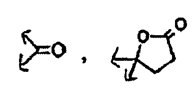 oder 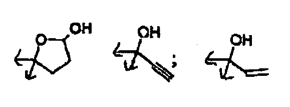 ist;
Y -CN, -CH₂-CH=CH₂, 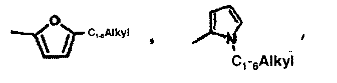 CHR₄C (O) Ar, CHR₄C(O)C₁₋₆Alkyl, CHR₄C(O)XAr oder CHR₄C(O)XC₁₋₆Alkyl ist;
worin
R₄ = OC₁₋₆Alkyl oder Aryl,
X = O oder S,
umfassend Umsetzen eines Steroid-Intermediates der Formel II 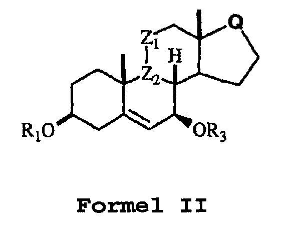 worin R₁ und R₃, Z₁, Z₂, R₂ und Q wie für Formel I sind;
mit einem nucleophilen Reagenz in Gegenwart eines Lewis-Säure-Katalysators.

2. Verbindung der Formel I, wie in Anspruch 1 beschrieben, worin:
worin
R₁ H oder -COR₂ ist;
R₂ C₁-C₆Alkyl oder C₁-C₆Alkoxy ist;
Z₁ CH₂ oder 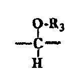 ist, worin OR₃ in der α-Konfiguration vorliegt;
R₃ H oder -COR₂ ist;
Z₂ -CH- ist; oder
Z₁ und Z₂ zusammengenommen werden können, um eine Kohlenstoff-Kohlenstoff-Doppelbindung zu bilden;
Q 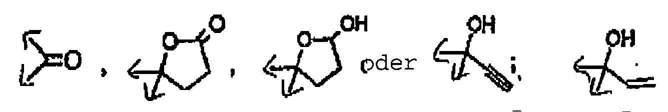 ist;
Y -CN, -CH₂-CH=CH₂, 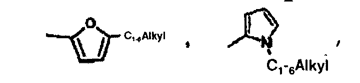 CHR₄C(O)Ar, CHR₄C (O)C₁₋₆Alkyl, CHR₄C(O) XAr oder CHR₄C (O)XC₁₋₆Alkyl ist;
worin R₄ = OC₁₋₆Alkyl oder Aryl,
X = O oder S.

3. Verfahren nach Anspruch 1, weiterhin umfassend die Stufen:
a) Umsetzen eines Ketosteroids der Formel 1 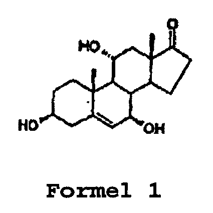 mit einem C₁-C₆Alkylchlorformiat oder Benzylchlorformiat oder einem Alkoxycarbonylbenztriazol in Gegenwart einer tertiären organischen Base unter Erhalt eines Tricarbonats der Formel 2, worin R C₁-C₆Alkyl oder Benzyl ist; 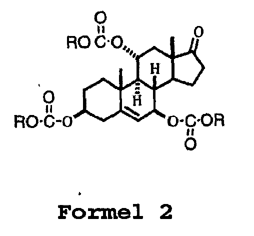
b) Umsetzen einer Triacylverbindung der Formel 2 mit einem 2-C₁₋₆-Alkylfuran in Gegenwart eines Lewis-Säure-Katalysators unter Erhalt einer Diacylesterverbindung der Formel 3; 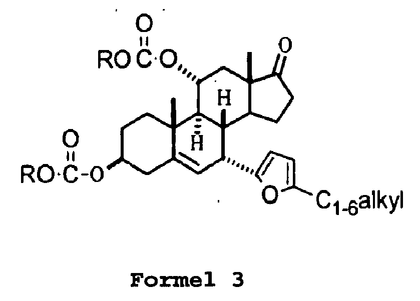
c) Hydrolysieren der Diacylesterverbindung der Formel 3 in Gegenwart einer Base unter Erhalt eines Dihydroxyesters der Formel 4; 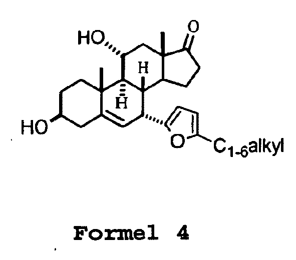
d) Umsetzen einer Verbindung der Formel 4 mit Acetylen in Gegenwart einer starken Base unter Erhalt einer acetylenischen Verbindung der Formel 5; 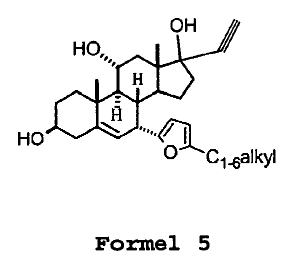
e) Umsetzen einer acetylenischen Verbindung der Formel VII mit Kohlenmonoxid in Gegenwart eines Rhodium-Katalysator-Liganden unter Erhalt eines Lactols der Formel 6; 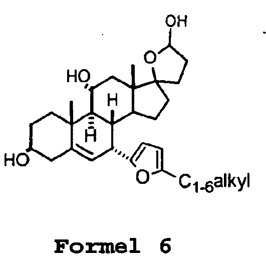
f) Oxidation eines Lactols der Formel 6 unter Erhalt eines Lactons der Formel 6a; 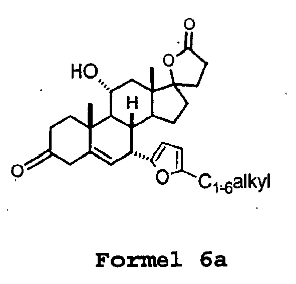
g) Isomerisieren der 4,5-Doppelbindung von 6a unter Erhalt eines Lactons der Formel 7; 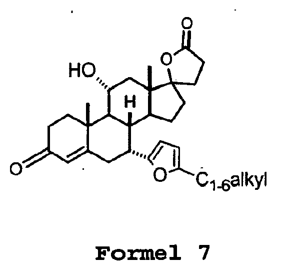
h) Bromieren, Ozonisieren, Oxidieren und Verestern einer Verbindung der Formel 7 unter Erhalt eines Esters der Formel 8; 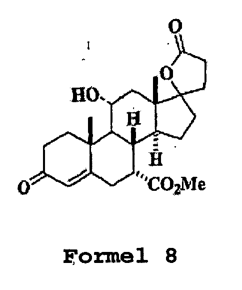
i) Dehydratisieren einer Verbindung der Formel 8 unter Erhalt eines Intermediates der Formel 9; 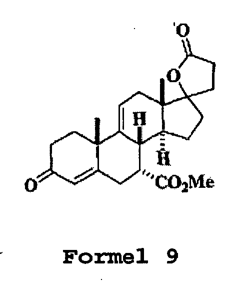
j) Oxidieren eines Dienons der Formel 9, wodurch Eplerenon (Formel 10) erhalten wird.

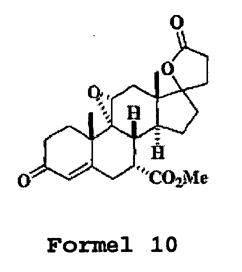

4. Verbindung der Formel 6a.

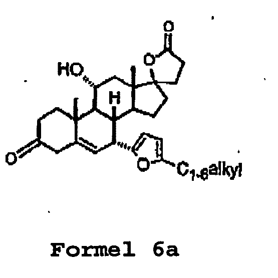

5. Verfahren gemäß Anspruch 1, umfassend die Stufen:
a) Umsetzen einer Verbindung der Formel 1 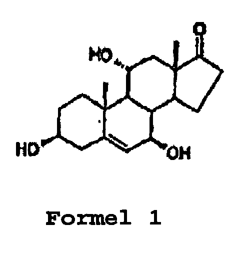 mit Acetylen, unter Erhalt einer Verbindung der Formel 11; 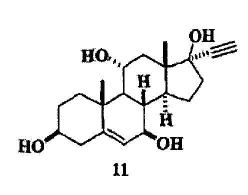
b) Acylieren einer Verbindung der Formel 11 unter Erhalt einer Verbindung der Formel 12; 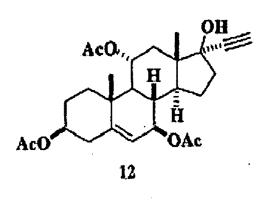
c) Hydroformylieren einer Verbindung der Formel 12 unter Erhalt einer Verbindung der Formel 13; 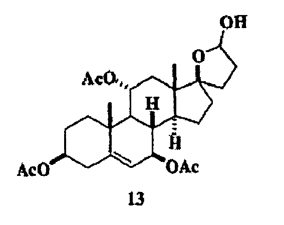
d) Oxidieren einer Verbindung der Formel 13 unter Erhalt einer Verbindung der Formel 14; 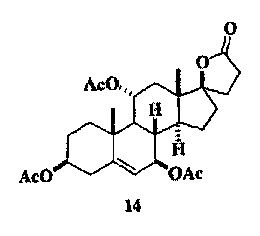
e) In-Kontakt-Bringen einer Verbindung der Formel 14 mit einem 2-Alkylfuran in Gegenwart einer Lewis-Säure unter Erhalt einer Verbindung der Formel 15; 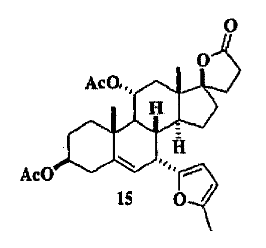
f) Hydrolysieren einer Verbindung der Formel 15 unter Erhalt einer Verbindung der Formel 16; 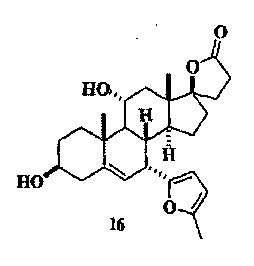
g) Oxidieren einer Verbindung der Formel 16 unter Erhalt einer Verbindung der Formel 17; 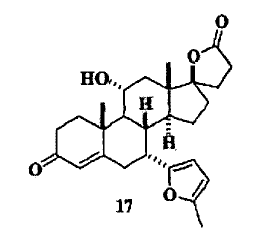
h) Umwandeln des'Furanrings einer Verbindung der Formel 17 in eine Methoxycarbonylverbindung der Formel 18; 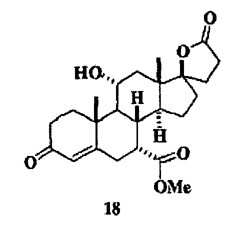
i) Umwandeln einer Verbindung der Formel 18 in einen Sulfonatester der Formel 19; 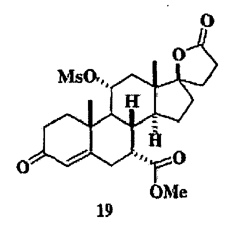
j) Eliminieren des Sulfonatesters der Formel 19 unter Erhalt einer Verbindung der Formel 9; 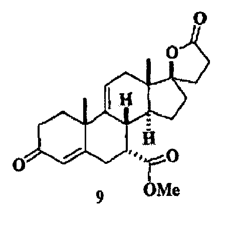
k) Oxidieren einer Verbindung der Formel 9, unter Erhalt einer Verbindung der Formel 10, Eplerenon.

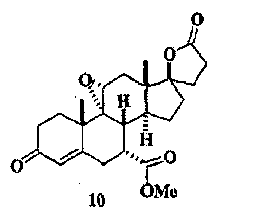

6. Verfahren zum Herstellen von Eplerenon gemäß Anspruch 5, weiterhin umfassend die Silylierung einer Verbindung der Formel 1 vor der Reaktion mit Acetylen, um ein silyliertes Intermediat zu erhalten und Entfernen der Silylgruppen während der Isolierung einer Verbindung der Formel 11.

## Revendications

1. Procédé de préparation de composés stéroïdes 7-substitués de Formule I, 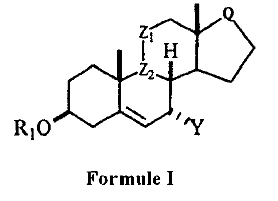 dans laquelle :
R₁ représente H ou -COR₂ ;
R₂ représentant alkyle en C₁-C₆ ou alcoxy en C₁-C₆ ;
Z₁ représente CH₂ ou 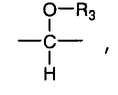 OR₃ étant dans la configuration α ;
R₃ représentant H ou -COR₂ ;
Z₂ représente -CH- ; ou
Z₁ et Z₂ peuvent être pris ensemble pour former une double-liaison carbone-carbone ;
Q représente 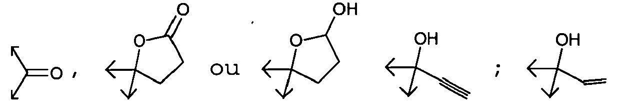
Y représente -CN, -CH₂-CH=CH₂, 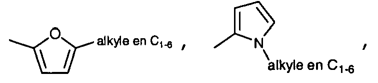 CHR₄C(O)Ar ; CHR₄C (O) (alkyle en C₁₋₆), CHR₄C (O) XAr ou CHR₄C(O)X(alkyle en C₁₋₆) ;
où R₄ = O (alkyle en C₁₋₆ ou aryle)
X = O ou S
procédé qui inclut la réaction d'un intermédiaire stéroïde de Formule II ; 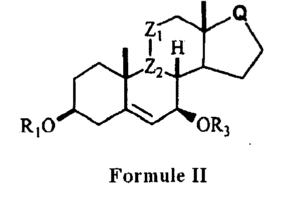 dans laquelle R₁ et R₃, Z₁, Z₂, R₂ et Q sont tels que définis pour la Formule I ;
avec un réactif nucléophile, en présence d'un acide de Lewis comme catalyseur.

2. Composé de Formule I tel que décrit dans la revendication 1, dans lequel :
R₁ représente H ou -COR₂ ;
R₂ représentant alkyle en C₁-C₆ ou alcoxy en C₁-C₆ ;
Z₁ représente CH₂ ou 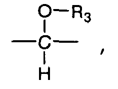 OR₃ étant dans la configuration α ;
R₃ représentant H ou -COR₂ ;
Z₂ représente -CH- ; ou
Z₁ ou Z₂ peuvent être pris ensemble pour former une double-liaison carbone-carbone ;
Q représente 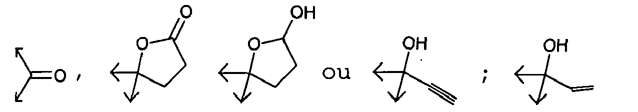
Y représente -CN, -CH₂-CH=CH₂, 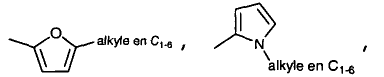 CHR₄C(O) Ar; CHR₄C(O) (alkyle en C₁₋₆), CHR₄C(O)XAr ou CHR₄C(O)X(alkyle en C₁₋₆) ;
où R₄ = O (alkyle en C₁₋₆ ou aryle)
X = O ou S.

3. Procédé selon la revendication 1, comprenant en outre les étapes de :
a) réaction d'un cétostéroïde de Formule 1 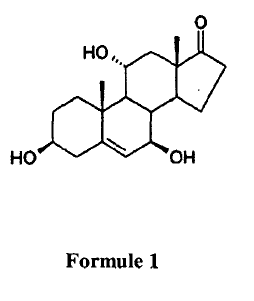 avec un chloroformiate d'alkyle en C₁-C₆ ou un chloroformiate de benzyle ou un alcoxycarbonylbenzotriazole, en présence d'une base organique tertiaire, pour donner un tricarbonate de Formule 2, dans laquelle R représente alkyle en C₁-C₆ ou benzyle ; 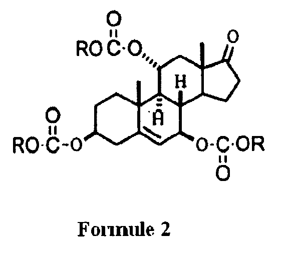
b) réaction d'un composé tri-acyle de Formule 2 avec un composé 2-(alkyle en C₁₋₆)furane, en présence d'un acide de Lewis comme catalyseur, pour donner un composé diacylester de Formule 3 ; 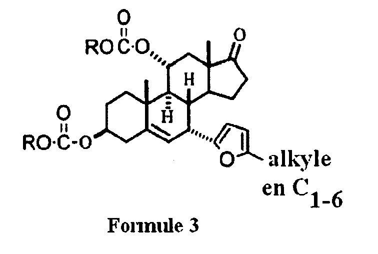
c) hydrolyse du composé diacylester de Formule 3, en présence d'une base, pour donner un dihydroxyester de Formule 4 ; 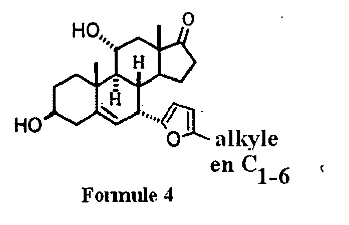
d) réaction d'un composé de Formule 4 avec l'acétylène, en présence d'une base forte, pour donner un composé acétylénique de Formule 5 ; 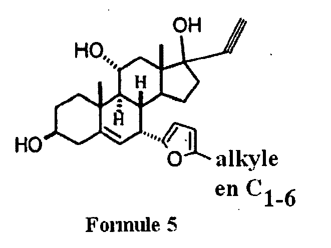
e) réaction d'un composé acétylénique de Formule XVII avec le monoxyde de carbone, en présence d'un catalyseur à base de rhodium/ligand, pour donner un lactol de Formule 6 ; 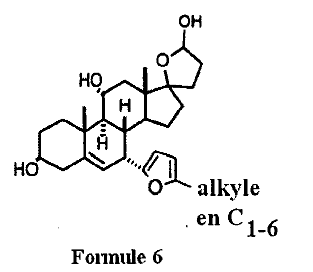
f) oxydation d'un lactol de Formule 6, pour donner une lactone de Formule 6a ; 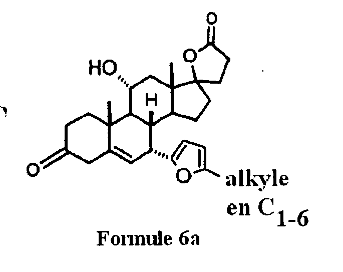
g) isomérisation de la double liaison en 4,5 de 6a, pour donner une lactone de Formule 7 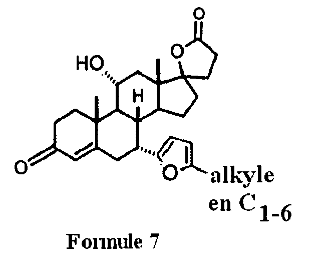
h) bromation, ozonisation, oxydation et estérification d'un composé de Formule **7**, pour donner un ester de Formule **8** ; 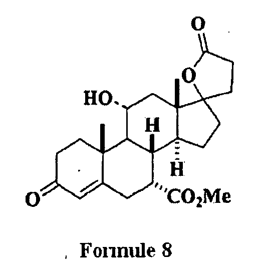
i) déshydratation d'un composé de Formule 8, pour donner un intermédiaire de Formule 9 ; 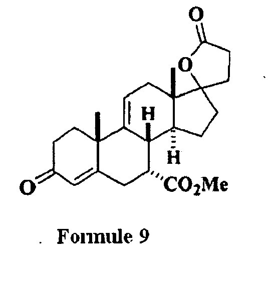
j) oxydation d'une dièneone de Formule 9, grâce à quoi l'éplérénone (Formule 10) 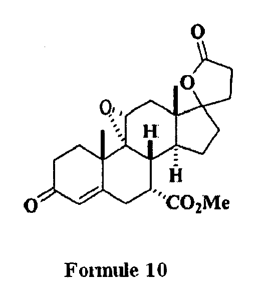 est obtenue.

4. Composé de Formule 6a.

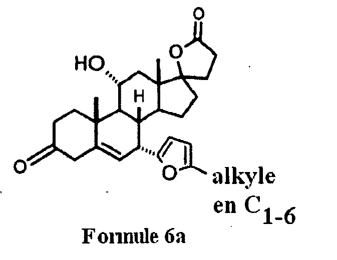

5. Procédé selon la revendication 1, comprenant en outre les étapes de :
a) réaction d'un composé de Formule 1 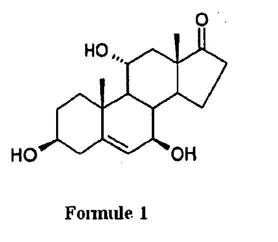 avec l'acétylène, pour donner un composé de Formule 11 ; 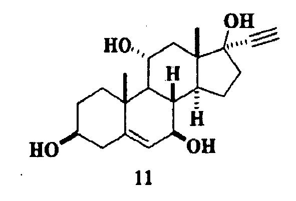
b) acylation d'un composé de Formule 11, pour donner un composé de Formule 12 ; 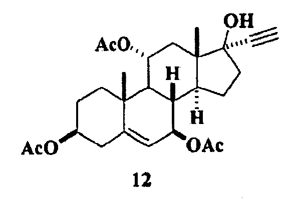
c) hydroformylation d'un composé de Formule 12, pour donner un composé de Formule 13 ; 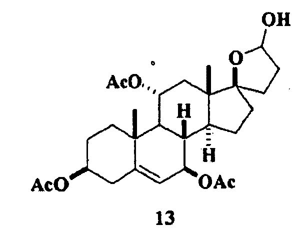
d) oxydation d'un composé de Formule 13, pour donner un composé de Formule 14 ; 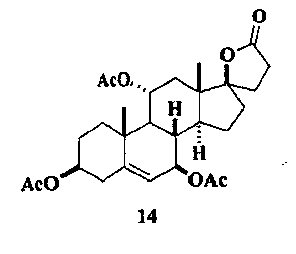
e) mise en contact d'un composé de Formule 14 avec un 2-alkylfurane, en présence d'un acide de Lewis, pour donner un composé de Formule 15 ; 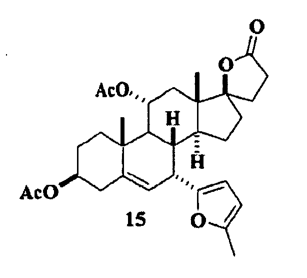
f) hydrolyse d'un composé de Formule 15, pour donner un composé de Formule 16 ; 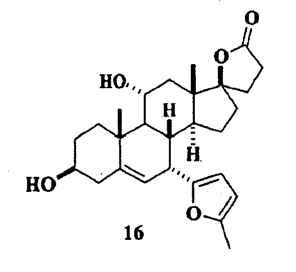
g) oxydation d'un composé de Formule 16, pour donner un composé de Formule 17 ; 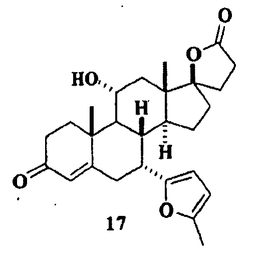
h) transformation du noyau furane d'un composé de Formule 17 en un composé méthoxycarbonyle de Formule 18 ; 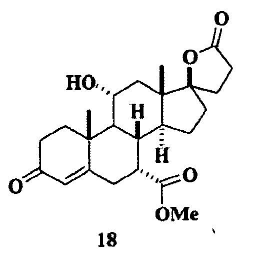
i) transformation d'un composé de Formule 18 en un ester sulfonique de Formule 19 ; 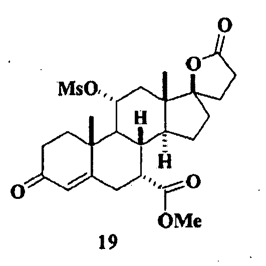
j) élimination de l'ester sulfonique de Formule 19, pour donner un composé de Formule 9 ; 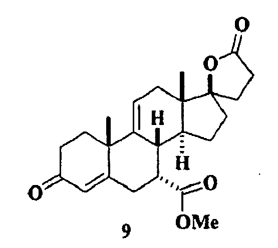
k) oxydation d'un composé de Formule 9, pour donner un composé de Formule 10, l'éplérénone.

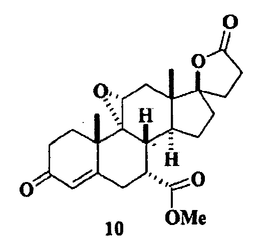

6. Procédé de préparation de l'éplérénone selon la revendication 5, comprenant en outre la silylation d'un composé de Formule 1 avant la réaction avec l'acétylène, pour donner un intermédiaire silylé et l'élimination desdits groupes silyle pendant l'isolement d'un composé de Formule 11.
